# EUROPEAN PATENT APPLICATION

(11) **EP 2 899 279 A1**
(43) Date of publication of application: **29.07.2015**
(21) Application number: 14305100.1
(22) Date of filing: 24.01.2014
(51) Int. Cl.: C12N 15/82, A01H 5/08

(54) **Resistance to gravepine fanleaf virus**

(71) Applicant: INSTITUT NATIONAL DE LA RECHERCHE AGRONOMIQUE, 75338 Paris Cedex 07 (FR); Centre National de la Recherche Scientifique, 75016 Paris (FR); VRIJE UNIVERSITEIT BRUSSEL, 1050 Brussel (BE); Vlaams Instituut voor Biotechnologie VZW, 9052 Gent (BE)
(72) Inventor: Ritzenthaler, Christophe, 67600 Selestat (FR); Demangeat, Gérard, 68150 Ostheim (FR); Hemmer, Caroline, 68000 Colmar (FR); Muyldermans, Serge, 1560 Hoeilaart (BE); Ackerer, Léa, 67550 Vendenheim (FR)
(74) Representative: Becker, Philippe

(57) **Abstract**

The invention relates to plant resistant to GFLV. More specifically, the invention relates to plants exhibiting VHH-mediated resistance to GFLV. The invention also relates to methods for conferring resistance to GFLV on plants or methods for producing plants resistant to GFLV. Furthermore, the invention relates to anti-GFLV VHHs molecules, nucleic acid sequences, expression cassettes, and recombinant vectors thereof.

## Description

### FIELD OF THE INVENTION

The invention relates generally to the field of agricultural biotechnology and plant diseases. In particular, the invention relates to plants resistant to *Grapevine fanleaf virus* (GFLV), an agent responsible for fanleaf disease, one of the most devastating diseases of commercial grapevine. More specifically, the invention relates to plants having an acquired VHH-mediated resistance to GFLV. The invention also relates to methods for conferring resistance to GFLV on plants and to methods for producing transgenic plants resistant to GFLV. Furthermore, the invention relates to VHH directed against GFLV antigen, amino acid sequence thereof, nucleic acid sequence, expression cassette, or recombinant vector thereof.

### BACKGROUND OF THE INVENTION

Fanleaf degeneration is a severe disease of grapevine that occurs worldwide and is caused by Nepoviruses. *Grapevine fanleaf virus* (GFLV) is the principal causal agent of this disease. Infected grapevine plants show dwarfed and/or deformed leaves with characteristic vein banding or mottling. The disease reduces vigor, yield by up to 80%, and fruit quality. It also reduces the lifespan of vineyards and can cause plant mortality (Andret-Link et al, 2004a; Martelli and Boudon-Padieu, 2006).

GFLV belongs to subgroup A of the genus *Nepovirus* in the family Secoviridae (Sanfaçon et al., 2009). GFLV is specifically transmitted from grapevine to grapevine by the ectoparasitic nematode *Xiphinema index* (Andret-Link et al., 2004b; Schellenberger et al., 2010, 2011). This nematode is able to survive in vineyard soils and retain GFLV for many years in the absence of host plants (Demangeat et al., 2005). As no natural sources of resistance to this virus has been identified in *Vitis vinifera,* the control of fanleaf disease is based on sanitary selection and soil disinfection using nematicides. Although the dissemination of the virus has been reduced by these measures, the control of GFLV in naturally infected vineyards is still inefficient. The use of nematicides is largely unsuccessful because of the nematode's ability to exist on detached grape roots deep in the soil profile (Demangeat et al., 2005; Esmenjaud and Bouquet, 2009), and is forbidden in many countries because of environmental toxicity. The sanitary selection requires the uprooting of infected plants followed by a fallow period of at least 7 years, which is difficult to implement for economical reasons especially in premium vineyards (Andret-Link et al., 2004a, Oliver and Fuchs, 2011).
Therefore, there is a strong need to develop virus-resistant grapevine varieties, which is likely to be the most effective, environmentally friendly and sustainable approach to control fanleaf disease.

Over the past 20 years, there has been enormous progress in virus resistance strategies based on genetic engineering. Transgenic plants have been created that express natural and engineered resistance genes, pathogen genes (pathogen-derived resistance) and antibodies against pathogen antigens (Nölke et al., 2004; Fuchs and Gonzalves, 2007; Nölke et al, 2009; Safamejad et al, 2011). In grapevine, the most popular strategy has been the expression of viral coat protein (CP) genes, thus achieving CP-mediated resistance (Laimer et al, 2009). Transgenic grapevine plants expressing CP showed different symptoms, but no resistance to the disease (Laimer et al, 2009).

Some approaches to overcome the virus infection were based on the use of antibodies, more particularly on recombinant antibodies directed against viral proteins to confer resistance against plant pathogens (Boonrod et al., 2004; Gargouri-Bouzid et al., 2006; Prins et al., 2005; Villani et al., 2005, Nölke et al., 2009, Safamejad et al., 2011). However, the success of these strategies was limited. This may be due to the fact that these strategies require functional expression of an antibody in the cytosol, where the viral pathogen is most vulnerable, while the correct folding of immunoglobulin domains in the reducing cellular cytoplasm has frequently failed (Safamejad et al., 2011). Even scFv antibodies, which do not need to assemble from separate subunits, have been described to find the cytosol inhospitable.

Consequently, there exists a high demand for novel efficient methods for controlling GFLV related disease, as well as for producing resistant plants, grapevine in particular, with increased resistance to GFLV.

### SUMMARY OF THE INVENTION

The present invention provides novel and efficient methods for producing plants resistant to *Grapevine fanleaf virus* (GFLV), or for increasing or inducing resistance to GFLV in plants. The present invention also relates to methods for treating an existing GFLV infection in plants, or for destructing GFLV in plants. The invention is based on the transfer or expression of single heavy chain variable domain immunoglobulins (VHH) in plants. As disclosed herein, the invention shows that VHH molecules can be effectively expressed in plant cells, in functional conformation, and can effectively and surprisingly confer up to 100% resistance to GFLV.

An object of the invention therefore relates to VHH-mediated GFLV resistant plants.

Another object of this invention relates to methods for conferring or increasing resistance to GFLV to a plant comprising introducing and/or expressing in said plant a VHH directed against a GFLV antigen.

The invention also relates to methods for producing a plant having resistance to GFLV, wherein the method comprises introducing or expressing an anti-GFLV VHH into the plant.

The invention also relates to methods for eliminating GFLV from an infected plant, the methods comprising introducing or expressing an anti-GFLV VHH into said plant or cells thereof.

As will be disclosed, the anti-GFLV VHH may be introduced or expressed in a plant, plant cell or plantlet by various techniques such as, preferably, by agrobacterium mediated transformation, biolistic techniques, virus-mediated infection, or grafting.

A further object of the invention relates to the use of a VHH directed against a GFLV antigen for inducing or increasing resistance of a plant to GFLV.

Most preferred VHH for use in the invention comprise particular CDR sequences as defined in the present application. More preferably, they comprise at least one CDR sequence as represented in SEQ ID NOs: 6 to 12, or a sequence having at least 80% similarity and/or identity to anyone of said CDR sequences. Such VHH per se, their coding nucleic acid molecules, as well as recombinant vectors comprising the same represent further objects of the invention. The invention also relates to cells or plants comprising a nucleic acid construct or a recombinant vector as defined above, as well as to seeds of such plants.

As will be discussed, the invention may be used in any plant, preferably a plant of the genus Vitis, such as without limitation *Vitis vinifera, Vitis vinifera, Vitis labrusca, Vitis riparia, Vitis aestivalis, Vitis rotundifolia, Vitis rupestris Vitis amurensis, Vitis coignetiae, Vitis vulpine* species, or any hybrid thereof.

### BRIEF DESCRIPTION OF THE DRAWINGS

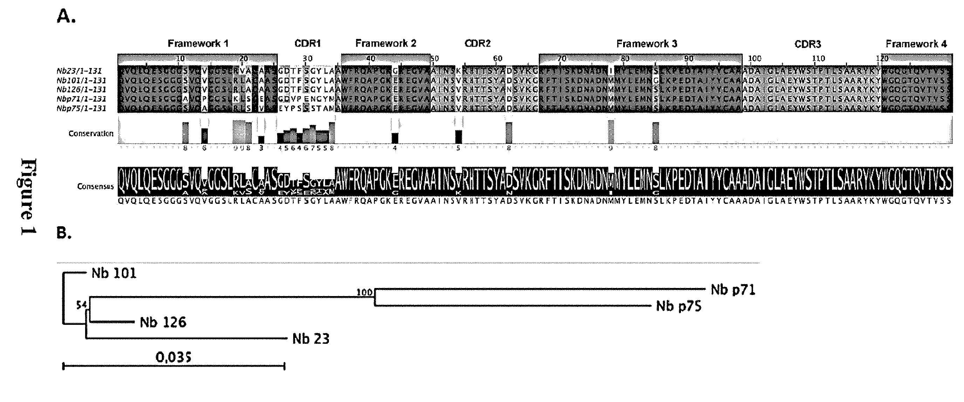

**Figure 1****: A. Primary sequence alignment of Nb23, Nb101, Nb126, Nbp71 and Nbp75** with identification of CDRs, Frameworks (FR) regions and consensus sequence. B. Dendrogram showing the grouping of VHH based on their genetic relatedness. Complete VHH sequences were used to generate the dendrogram.

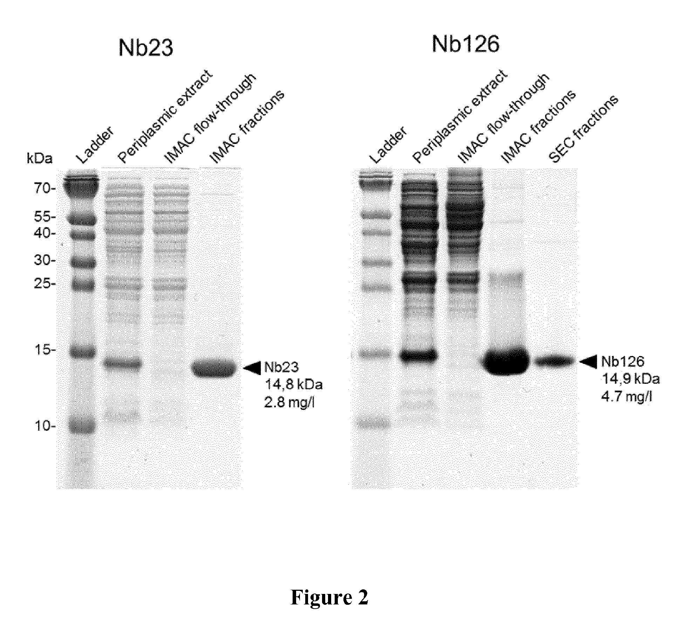

**Figure 2****: Coomassie stained denaturing polyacrylamide gel** illustrating the purification by immobilized metal ion affinity chromatography (IMAC) followed by size exclusion chromatography (SEC) **of Nb23** (left panel) **and Nb126** (right panel). Arrowheads point to His-tagged Nb23 and Nb 126. Molecular weight and average yields in mg/l of bacterial culture are indicated for each Nb.

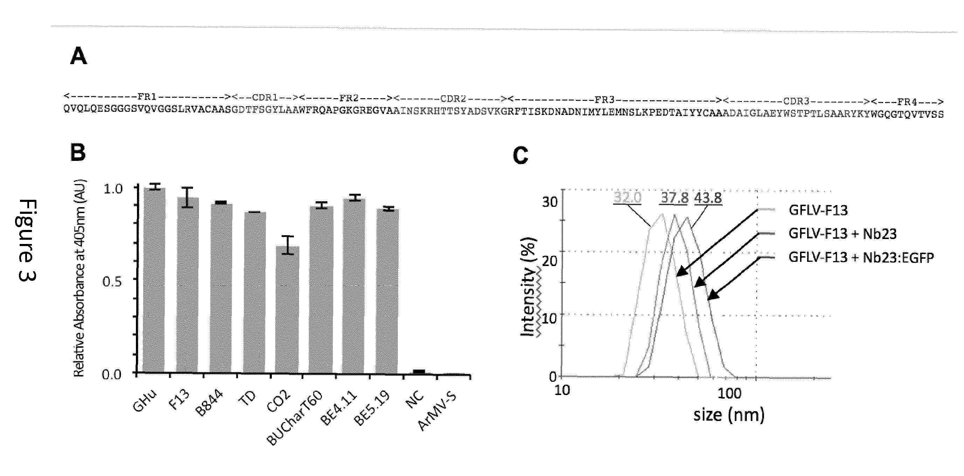

**Figure 3****: Identification and in vitro characterization of nanobody Nb23. (A) Amino acid sequence of Nb23** (131 amino-acids). Frameworks (FR) are indicated and complementarity determining regions (CDRs) are shaded. (B) Nb23 was tested for antigen specificity by DAS-ELISA against a collection of GFLV isolates and ArMV-S from infected *Chenopodium quinoa.* All GFLV isolates (GFLV-GHu, -F13, -B844, -TD, -CO2, -BUChardT60, -BE4.11 and -BE5.19) showed significant binding response whereas ArMV-S failed to be recognized. Healthy plants were used as negative control (NC). Bars show representative values of experimental duplicates. (C) Dynamic light scattering (DLS) analyses of GFLV-F13 alone (left curve; average hydrodynamic diameter of 32 nm) or as a complex to Nb23 (middle curve; average hydrodynamic diameter of 37.8 nm) or Nb23: EGFP (right curve; average hydrodynamic diameter of 43.8 nm). Average hydrodynamic diameters in nm are indicated above each curve. Note the increase in GFLV-F13 size upon addition of Nb23 and Nb23: EGFP.

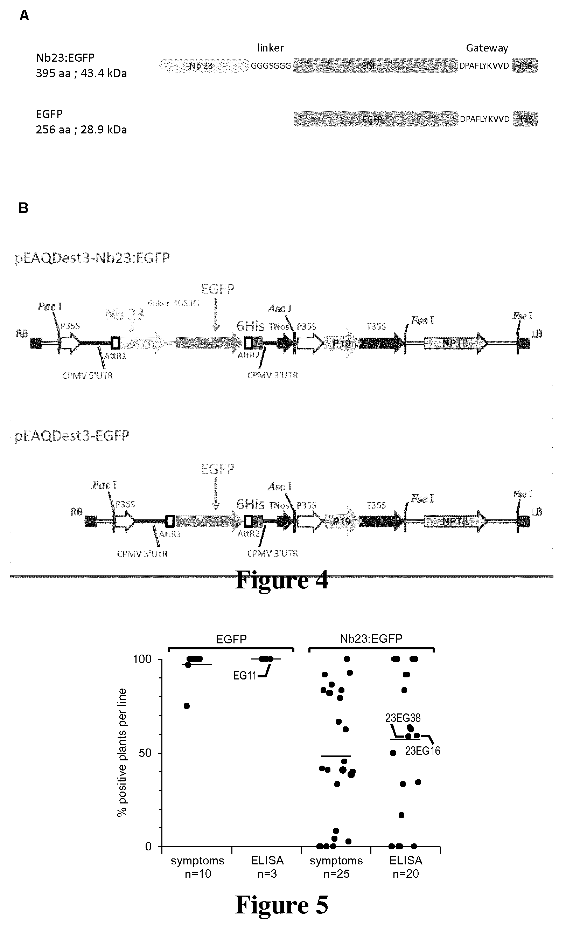

**Figure 4****. Primary structure of Nb23: EGFP and EGFP constructs and vectors used for plant transformation. A.** Primary structure of Nb23: EGFP and EGFP constructs. Numbers of residues and molecular mass are given for each construct. A Gly₃SerGly₃ linker was introduced between Nb23 and EGFP sequences. Extra-residues at the C-terminus resulting from Gateway cloning are indicated. **B.** Schematic representation of the vector T-DNA regions used for transformation
**Figure 5****: Evaluation of T1 transgenic *N. benthamiana* lines challenged with GFLV-GHu.** A total of 10 EGFP and 25 Nb23: EGFP T1 lines, each consisting of 12 to 39 segregating plants, were mechanically inoculated with 300 ng of purified GFLV-GHu and infection monitored by symptoms assessment over 28 days and by anti-GFLV DAS-ELISA analysis at 28 dpi. Each dot on the graph corresponds to the percentage of infection per plant line. The numbers of lines analyzed for symptoms and by DAS-ELISA are indicated by asterisks. Horizontal bars indicate mean percentage values. Note that infection rates as determined by ELISA range from 0 to 100 % for lines Nb23: EGFP compared to 100 % for lines EGFP. The EGFP and Nb23: EGFP T1 lines selected to obtain T2 progeny are indicated.

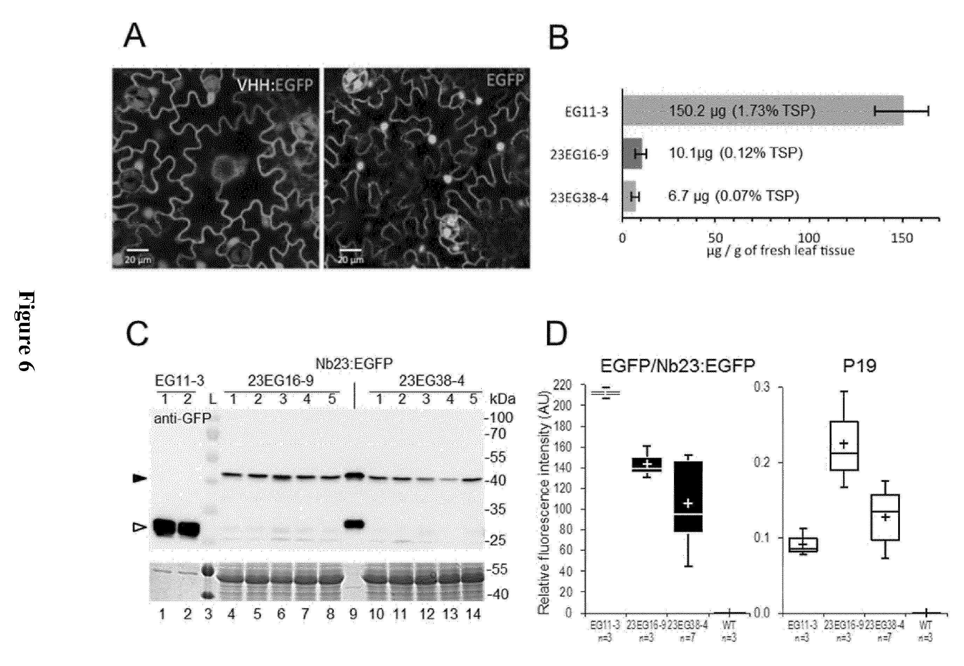

**Figure 6****: Characterization of homozygous T2 transgenic lines EG11-3, 23EG16-9 and 23EG38-4.** A. Nb23: EGFP expressed from lines 23EG16-9 and 23EG38-4 (left) is found in the cytoplasm and nucleus of leaf epidermal cells, similarly to EGFP expressed from line EG11-3 (right). **B.** Quantification of Nb23: EGFP and EGFP from lines EG11-3, 23EG16-9 and 23EG38-4. Fluorescence was quantified in soluble leaf extracts from transgenic lines and compared to fluorescence produced from known amounts of purified Nb23: EGFP from *E*. *coli.* TSP: total soluble proteins **C.** Immunoblot analysis of Nb23: EGFP and EGFP produced by lines EG11-3, 23EG16-9 and 23EG38-4. Total soluble proteins from leaves of EG11-3 (0.25 mg fresh tissue equivalent, lanes 1, 2), 23EG16-9 (7.5 mg fresh tissue equivalent, lanes 4 to 8) and 23EG38-4 (7.5 mg fresh tissue equivalent, lanes 10 to 14) plants were probed with anti-GFP antibodies. Semi-purified Nb23: EGFP expressed from *E*. *coli* was used as size control (40 ng, lane 9) for Nb23: EGFP (black arrowhead). Open arrowhead points to EGFP. Bottom panel: Equal loading control: Coomassie blue straining of the membrane. Lane 3: ladder with protein size indicated at the right. D. RTqPCR analysis of lines EG11-3, 23EG16-9 and 23EG38-4. Relative accumulation of mRNA encoding EGFP and Nb23 :EGFP (black) or P 19 (white) determined by RTqPCR. Plus sign corresponds to mean value and whiskers to lowest or highest values within 1.5 interquartile range of the lower or higher quartile. Thick dash: maximum outlier value. Note the different scaling for mRNA encoding P19 and those encoding EGFP or Nb23: EGFP.

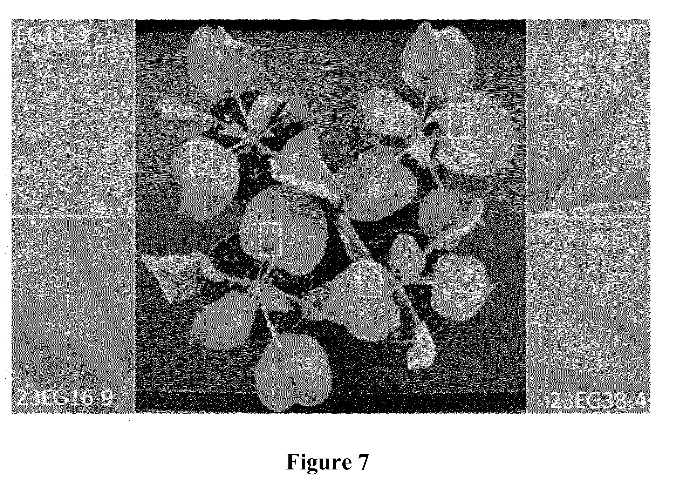

**Figure 7****. Assessment of GFLV-GHu symptoms of mechanically inoculated 23EG16-9, 23EG38-4, EG11-3 T2 lines and WT *N*. *benthamiana* at 7 dpi.** All WT and EGFP lines showed mosaic symptoms on systemic leaves whereas 23EG16-9 and 23EG38-4 lines remained asymptomatic. Inoculations were performed with 300 ng of purified GFLV-GHu.

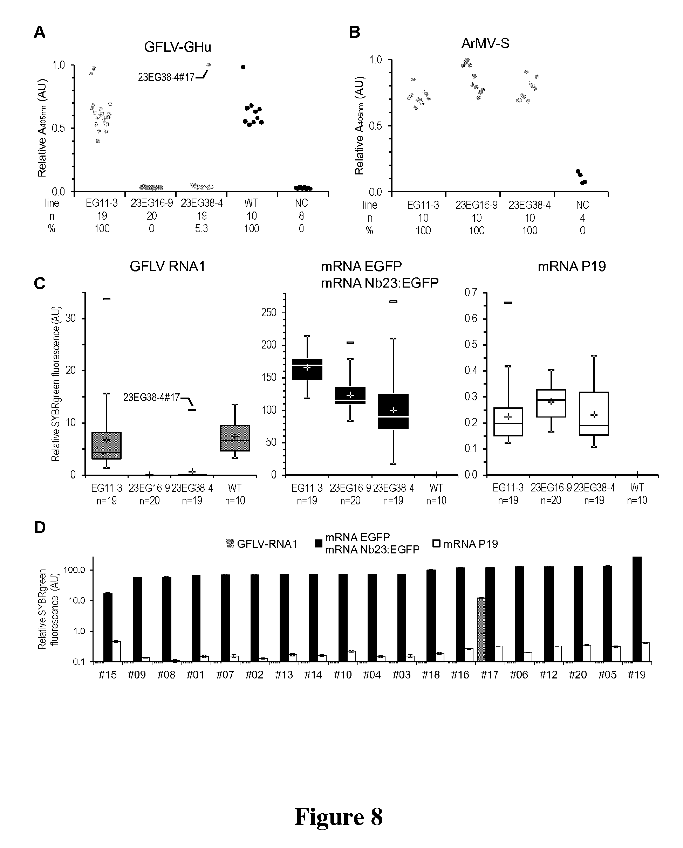

**Figure 8****: Evaluation of transgenic *N. benthamiana T2* lines 23EG16-9 and 23EG38-4 to infection by GFLV-GHu and ArMV-S.** Anti-GFLV (A) and anti-ArMV (B) DAS-ELISA performed at 21 dpi on upper uninoculated leaves from EG11-3, 23EG16-9, 23EG38-4 and WT plants. Each spot corresponds to a single sample and represents the mean relative absorbance at 405 nm of experimental duplicates. Number of plant tested (n) and percentages of infections (%) are provided below each column. C. RTqPCR analysis of the same samples as in A. Relative accumulation of RNA1 transcripts (grey), EGFP and Nb23:EGFP mRNAs (black) or P19 mRNA (white) determined by RTqPCR. Plus sign corresponds to mean value and whiskers to lowest or highest values within 1.5 interquartile range of the lower or higher quartile. Thick dash: maximum outlier value. Note that in line 23EG38-4, one plant (#17) revealed GFLV-positive by ELISA and RTqPCR. **D.** Relative accumulation of RNA1 transcripts (grey), and mRNA encoding EGFP and Nb23: EGFP (black) or P19 (white) determined by RTqPCR in individual plants from line 23EG38-4. Error bars show standard deviation of experimental triplicates. RNA1 is clearly detected in plant #17 only. All inoculations were performed with 300 ng of purified GFLV-GHu or ArMV-S.

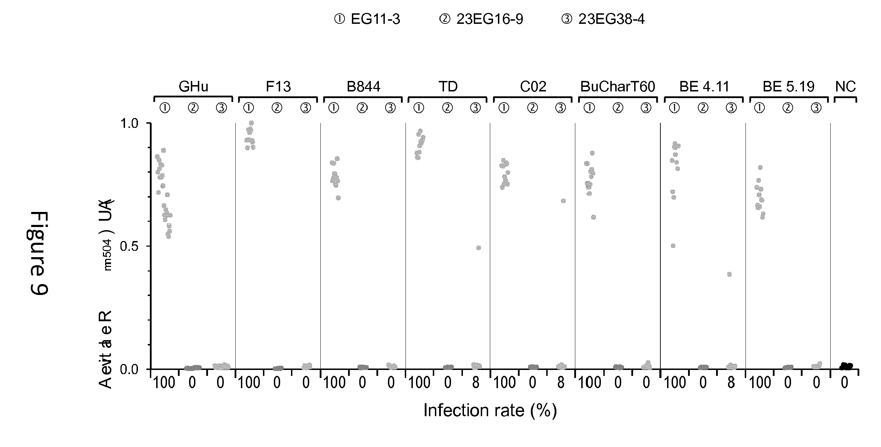

**Figure 9****: Evaluation of the resistance of T2 lines 23EG16-9 and 23EG38-4 to infection by various GFLV isolates.** EG11-3, 23EG16-9 and 23EG38-4 plants were mechanically inoculated with saps from C. *quinoa* plants infected with GFLV isolates-GHu, -F13, -B844, -TD, -CO2, -BUChardT60, -BE4.11 and -BE5.19 and apical non-inoculated leaves analyzed by DAS-ELISA at 21 dpi. Each dot corresponds to a single sample and represents the mean relative absorbance at 405 nm of experimental duplicates. ①② and and ③ correspond to lines EG11-3, 23EG16-9 and 23EG38-4, respectively. Non-inoculated plants were used as negative control (NC). For each set of inoculation, a total of 12 plants per line were tested except for GFLV-GHu (24 plants per line). Percentages of infections are indicated below each column (%). Note that EG11-3 plants are fully susceptible to GFLV, whereas line 23EG16-9 is 100% resistant to all GFLV isolates. Altogether only three plants out of 108 tested positive for GFLV in line 23EG38-4.

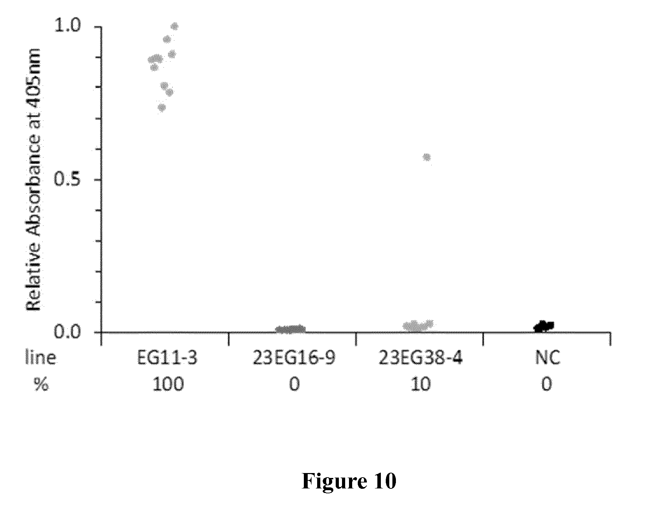

**Figure 10****: Evaluation of resistance of T2 lines 23EG16-9 and 23EG38-4 to infection with viral RNA.** EG11-3, 23EG16-9 and 23EG38-4 plants were mechanically inoculated with 360 ng of GFLV-GHu viral RNA and apical non-inoculated leaves analyzed by DAS-ELISA at 21 dpi. Relative absorbance values of individual samples are indicated by dots. Percentages of infections are indicated below each column (%). 10 plants were tested per transgenic line. Note that line 23EG16-9 is fully resistant to viral RNA whereas only one plant tested positive for GFLV in line 23EG38-4.

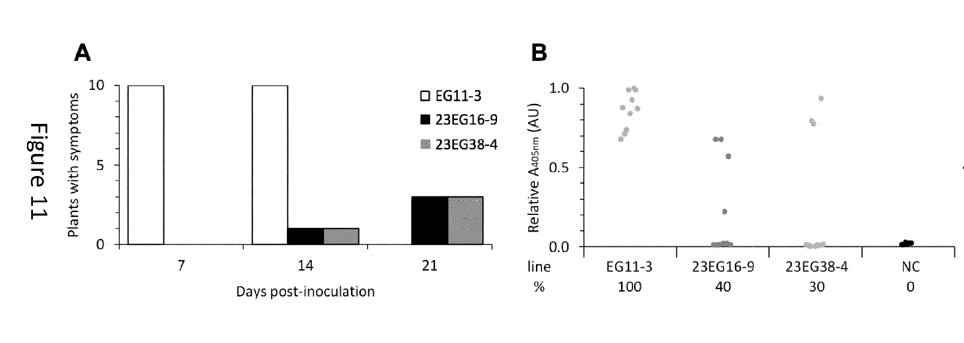

**Figure 11****: Evaluation of** the **resistance of T2 lines 23EG16-9 and 23EG38-4 to GFLV at high pressure of inoculum.** EG11-3, 23EG16-9 and 23EG38-4 plants were mechanically inoculated with 3 µg of GFLV-GHu. A. Number of plants with symptoms at 7, 14 and 21 dpi. **B.** GFLV detection by DAS-ELISA at 21 dpi. Relative absorbance values of individual samples are indicated for each plant line. 10 plants were tested for each set of inoculum. Percentages of infection are indicated below each column (%). Note the delay in symptoms appearance and the reduced number of infected plants in lines 23EG16-9 and 23EG38-4 compared to EG11-3

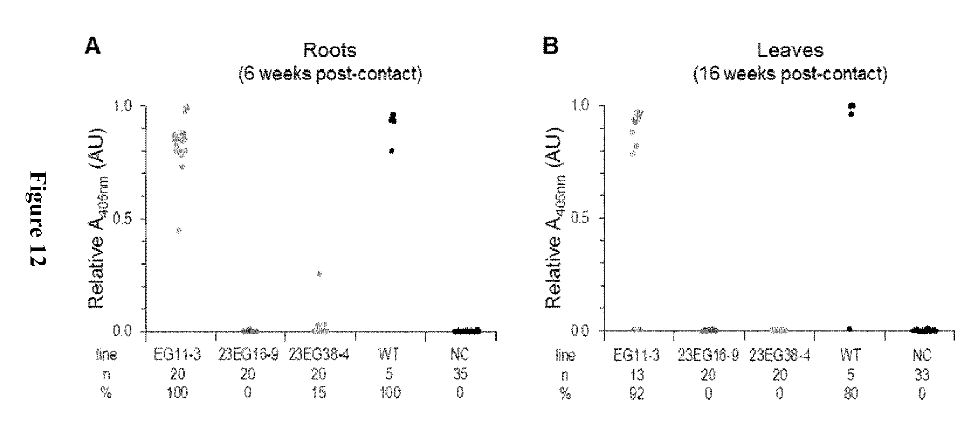

**Figure 12****. Evaluation of the resistance of T2 lines 23EG16-9 and 23EG38-4 to GFLV transmission by *X. index.*** EG11-3, 23EG16-9, 23EG38-4 and WT plants were grown in contaminated soil containing viruliferous nematodes. Plants of each genotype maintained in contact with aviruliferous *X. index* were included as negative control (NC). A. GFLV-DAS-ELISA analysis of roots 6 weeks post-contact with nematodes. **B.** GFLV-DAS-ELISA analysis of leaves from the same plants 16 weeks after initial contact with nematodes. Relative absorbance values of individual samples are indicated for each plant line. Number of plant tested (n) and percentage of infection (%) are provided below each column. Note the low OD and frequency of infection in roots and the absence of systemic infection in lines 23EG16-9 and 23EG38-4

### DETAILED DESCRIPTION OF THE INVENTION

The present invention relates to novel compositions and methods for conferring or increasing resistance to GFLV to plants based on the expression or transfer of a VHH directed against GFLV. The invention also relates to methods for producing such plants, novel VHH molecules, and the uses thereof. The inventors have identified particular VHH molecules and shown that expression thereof can confer specific and broad spectrum resistance to GFLV in plants, demonstrating the large utility and advantages of the invention. In particular, the invention surprisingly shows that a 100% resistance to GFLV can be obtained by VHH expression in plants.

The present disclosure will be best understood by reference to the following definitions:

### Definitions

The term VHH (or nanobody, Hamers-Casterman et al., 1993) within the context of the present invention designates a single chain polypeptide consisting essentially of three CDRs (complementarity-determining regions CDR1, CDR2 and CDR3) separated by four FR domains (for Framework regions) and essentially devoid of a light chain or a constant domain.

The term *"plant"* as used herein refers to wild or cultivated plants and includes without limitation transgenic, non-transgenic, hybrid, or grafted plants, or any combination(s) thereof.

The term *"grapevine plant"* or *"Vitis genus plant"* as used herein are interchangeable and refer to the common name grapevine plant.

The term *"GFLV"* or *"Grapevine fanleaf virus"* as used herein are interchangeable and refer to any GFLV isolate.

As used herein, the term *"expression"* or *"expressing"* refers to the production of a polypeptide as a result of transcription and/or translation of a DNA or RNA molecule. Expression may be obtained by techniques known per se in the art such as, without limitation, by genetic means (e.g., introduction of a nucleic acid encoding the polypeptide in a plant, cells thereof, or an ancestor thereof). Expression may be transient or permanent, regulated, inducible or constitutive.

The term *"deliver"* in relation to the VHH as used herein, refers to any method that allows the VHH of the invention to be produced, introduced, delivered or present in a plant cells. This includes transgenesis, transient expression, expression via viral vectors, grafting, agrocapsule or microcapsule delivery (http://www.agrosavfe.be/technology.htm, or patent applications WO201350594 or EP2555614).

The term *"identity"* in relation to a protein sequence as used herein, refers to the degree of correspondence between two amino-acid sub-sequences (no gaps between the sequences). In other terms, it is the extent, expressed as a percentage, to which two protein sequences have the same amino acid at equivalent positions. The % identity can be determined by known computer programs such as BLAST, FASTA, etc.

The term *"similarity"* in relation to a protein sequence as used herein, refers to the extent to which the amino acid sequences of two proteins have identical or functionally similar amino acids at equivalent positions, usually expressed as a percentage. The degree of resemblance between two amino-acid sequences when they are compared depends on their nature. For example, residues Aspartic acid and Glutamic acid are considered to be functionally similar (French and Robson, 1983). Preferably, the similar sequences are identified by alignment using, for example, computer programs known in the art (BLAST, FASTA, etc.).

As used herein, the term *"Transgenic plant"* refers to a plant wherein all or part of the cells of said plant contain a transgene (e.g., a nucleic acid construct). Transgenic plants may be generated from a plant cell containing the transgene, so that all cells in the generated plant contain the transgene.

As used herein, the term *"GFLV antigen"* refers to any *Grapevine fanleaf virus* antigenic determinant or epitope present in a GFLV viral particle. GFLV antigen may be of any nature, e.g., of a peptide, polypeptide, nucleic acid, lipid or glucoside, for instance. Typically, the GFLV antigen is of peptidic nature. The GFLV antigen may be exposed on a GFLV particle or by a GFLV-infected cell, e.g. a structural protein or an epitope thereof, or not exposed, e.g. a non-structural protein or nucleoprotein, or an epitope thereof. As a particular example, the GFLV antigen may be an antigen present in/on the capsid of a GFLV, such as a coat protein sub-unit of the capsid. A VHH "against" or "directed against" a GFLV antigen, or an "anti-GFLV" VHH, designates a VHH that is able to selectively bind to a GFLV antigen or particle, preferably with affinity and/or specificity. In a particular embodiment, an "anti-GFLV" VHH, designates a VHH that is able to bind to a GFLV antigen or particle and that does not bind a distinct virus with high affinity.

The term *"transiently"* expressed as used herein refers to the temporary expression of a polypeptide (such as a VHH), as opposed to a permanent expression thereof. Expression may be transient as a result of the use of a particular promoter (e.g., a plant development-stage-specific promoter, or an inducible promoter) or of the type of vector used to deliver the VHH, or of the method of delivering the VHH to a plant (e.g., by spraying, mechanical inoculation or certain virus-mediated infection).

The term *"construct"* as used herein refers to a non-naturally occurring nucleic acid molecule comprising a coding sequence operably linked to a promoter functional in plant cells. The construct may be DNA or RNA, single- or double-stranded. It is typically a double-stranded DNA.

The introduction of a nucleic acid in a plant or cell by *"viral-mediated infection"* refers to the exposure of said plant or cell to a virus comprising, in its genome, said nucleic acid, resulting in infection of the plant or cell and introduction of the nucleic acid. The virus used has tropism for plant and is preferably innocuous.

The term increased or induced *"resistance"* refers to any improvement in the ability of a plant or cell to resist to an exposure or the presence of a GFLV virus. This includes a reduction or suppression of GFLV virus titer in said plant or cell, a reduction or suppression of GFLV virus accumulation, a reduction or suppression of GFLV virus transmission from plant to plant (e.g., by nematodes vectors), or a reduction or suppression of an effect or symptom of GFLV infection, such as cell/plant degeneration or death. More particularly, induced or increased GFLV resistance can lead to an increased lifespan of the plant, can reduce fruit loss or increase fruit quality.

### VHH molecules of the invention

The present invention discloses novel anti-GFLV VHH polypeptides and nucleic acid molecules, having high affinity for GFLV and which may be used to confer resistance to GFLV in plants.

In general, the specificity of an antibody is the structural complementarity between the combining site of the antibody and the antigenic determinant. Combining sites of the antibody are made of residues derived primarily from hypervariable regions or complementarity determining regions (CDR). Occasionally, residues from non-hypervariable regions or regions "framework" ("framework", FR) can influence the overall structure of the domain and therefore the combining site.

In the context of the invention, the term "nanobodies", "nanobody", "VHH", "VHH antibody fragment" or "single-domain antibody" are used interchangeably and designates single heavy chain variable domain immunoglobulins. VHH generally comprises (or consists essentially of) three CDRs (complementarity-determining regions CDR1, CDR2 and CDR3) separated by four FR (Framework Regions) domains, and essentially devoid of a light chain. VHHs therefore have the following structure: FR1-CD1-FR2-CD2-FR3-CD3-FR4. VHHs have been found naturally in camelids (camels, dromedaries, llamas, guanaco or alpacas). Preferably, the VHHs of the invention are synthetic molecules produced by recombinant or chemical technologies. VHHs present high epitope specificity and affinity. They are about ten times smaller than conventional IgG molecules. They are single-chain polypeptides, very stable, resisting extreme pH and temperature conditions. Moreover, they can resist to the action of proteases.

The present invention discloses the isolation and characterization of particular VHHs directed against a common GFLV antigen and having the ability to induce GFLV-resistance in plants. These specific VHHs, designated hereinafter Nb126, Nb101, Nb23, Nbp75 and Nbp71, bind to a same antigenic molecule, cross-react to different GFLV isolates, have a very strong affinity for GFLV and, when delivered or expressed in a plant, can confer full resistance to GFLV. These molecules, as well as the binding domains thereof, therefore represent very potent means to treat plants against GFLV. These VHH molecules further demonstrate the ability to induce or increase GFLV resistance using VHHs.

More specifically, the amino acid sequences of 5 specific VHHs of the invention are disclosed in SEQ ID NO: 1 to 5 and summarized in the following Table.

| **SEQ ID number** | **VHH** |
|---|---|
| SEQ ID NO: 1 | Nb126 |
| SEQ ID NO: 2 | Nb101 |
| SEQ ID NO: 3 | Nb23 |
| SEQ ID NO: 4 | Nbp75 |
| SEQ ID NO: 5 | Nbp71 |

Figure 1 shows the alignment of amino acid sequences SEQ ID NO: 1-5, and functional domains thereof. The specific CDR domains of each of these VHH are identified in the following table:

| **Corresponding VHH CDR** | **SEQ ID number** | **Amino acid sequence** |
|---|---|---|
| Nb126 CDR1 | SEQ ID NO: 6 | GDTFSGYLAA |
| Nb126 CDR2 | SEQ ID NO: 7 | AINSVRHTTSYANSVKG |
| Nb126 CDR3 | SEQ ID NO: 8 | ADAIGLAEYWSTPTLSAARYKY |
| Nb101 CDR1 | SEQ ID NO: 6 | GDTFSGYLAA |
| Nb101 CDR2 | SEQ ID NO: 9 | AINSVRHTTSYADSVKG |
| Nb101 CDR3 | SEQ ID NO: 8 | ADAIGLAEYWSTPTLSAARYKY |
| Nb23 CDR1 | SEQ ID NO: 6 | GDTFSGYLAA |
| Nb23 CDR2 | SEQ ID NO: 10 | AINSKRHTTSYADSVKG |
| Nb23 CDR3 | SEQ ID NO: 8 | ADAIGLAEYWSTPTLSAARYKY |
| Nbp75 CDR1 | SEQ ID NO: 11 | EYPSSSTAMA |
| Nbp75 CDR2 | SEQ ID NO: 9 | AINSVRHTTSYADSVKG |
| Nbp75 CDR3 | SEQ ID NO: 8 | ADAIGLAEYWSTPTLSAARYKY |
| Nbp71 CDR1 | SEQ ID NO: 12 | GDVPENGYMA |
| Nbp71 CDR2 | SEQ ID NO: 9 | AINSVRHTTSYADSVKG |
| Nbp71 CDR3 | SEQ ID NO: 8 | ADAIGLAEYWSTPTLSAARYKY |

Strikingly, all of the active VHHs share the same CDR3 sequence (SEQ ID NO: 8). Furthermore, they exhibit very similar CDR2 domains, only 2 positions (out of 17 residues) displaying a variability. In this regard, the following amino acid sequence (SEQ ID NO: 13) is the consensus amino acid sequence of CDR2 sequences identified:
Ala-Ile-Asn-Ser-X₁-Arg-His-Thr-Thr-Ser-Tyr-Ala-X2-Ser-Val-Lys-Gly (SEQ ID NO: 13)
wherein :
- X₁ is a lysine or a valine residue, preferably a Val and
- X₂ is an aspartate or an asparagine residue; preferably an Asp.

CDR1 domains also present a level of structural similarity. More particularly, the CDR1 all respond to the following formula (SEQ ID NO: 14):
X₁-X₂-X₃-X₄-X₅-X₆-X₇-X₈-X₉-Ala (SEQ ID NO: 14)
   wherein :
   - X₁ is a glycine or a glutamate residue, preferably a Gly;
   - X₂ is an aspartate or a tyrosine residue, preferably an Asp ;
   - X₃ is a threonine or a valine or a proline residue, preferably a Thr;
   - X₄ is a phenylalanine or a proline or a serine residue, preferably a Phe;
   - X₅ is a serine or a glutamate residue; preferably a Ser;
   - X₆ is a glycine or an asparagine or a serine residue, preferably a Gly;
   - X₇ is a tyrosine or a glycine or a threonine residue, preferably a Tyr;
   - X₈ is a leucine or a tyrosine or an alanine residue, preferably a Leu; and
   - X₉ is an alanine or a methionine residue; preferably an Ala.

Accordingly, in a particular embodiment, the invention relates to a polypeptide, preferably a VHH, comprising the amino acid sequence of SEQ ID NO: 8 or a sequence having at least 80% similarity or identity to SEQ ID NO: 8, preferably at least 90%, 92%, 94%, 95%, 96%, 97%, 98%, 99% or more amino-acid sequence similarity or identity to SEQ ID NO: 8.

Another particular embodiment, the invention relates to a polypeptide, preferably a VHH, comprising the amino acid sequence of SEQ ID NO: 13 wherein X₁ is a lysine or a valine residue and X₂ is an aspartate or an asparagine residue, or a sequence having at least 80% similarity or identity to SEQ ID NO: 13, preferably at least 90%, 92%, 94%, 95%, 96%, 97%, 98%, 99% or more amino-acid sequence similarity or identity to SEQ ID NO: 13. In specific and preferred embodiments, the invention relates to a polypeptide comprising the sequence of SEQ ID NO: 7, 9 or 10 or a sequence having at least 80% sequence identity thereto, preferably at least 90%, 92%, 94%, 95%, 96%, 97%, 98%, 99% or more amino-acid sequence identity thereto.

Another particular embodiment, the invention relates to a polypeptide, preferably a VHH, comprising the amino acid sequence of SEQ ID NO: 14 as defined above or a sequence having at least 80% similarity or identity to SEQ ID NO: 14, preferably at least 90%, 92%, 94%, 95%, 96%, 97%, 98%, 99% or more amino-acid sequence similarity or identity to SEQ ID NO: 14. In specific and preferred embodiments, the invention relates to a polypeptide comprising the sequence of SEQ ID NO: 6, 11 or 12 or a sequence having at least 80% sequence identity thereto, preferably at least 90%, 92%, 94%, 95%, 96%, 97%, 98%, 99% or more amino-acid sequence identity thereto.

In preferred embodiments, the polypeptides of the invention are VHH polypeptides comprising three CDR domains, at least one of said CDRs domains having a sequence selected from SEQ ID NO: 8, SEQ ID NO: 13 or SEQ ID NO: 14 as defined above, or a sequence having at least 80% similarity or identity to SEQ ID NO: 8, SEQ ID NO: 13 or SEQ ID NO: 14, preferably at least 90%, 92%, 94%, 95%, 96%, 97%, 98%, 99% or more amino-acid sequence similarity or identity thereto. In this regard, in a preferred embodiment, the invention is related to a VHH comprising at least one, preferably at least two, and still more preferably the three following CDR sequence(s):
**(a).**CDR1: X₁-X₂-X₃-X₄-X₅-X₆-X₇X₈-X₉-Ala (SEQ ID NO: 14)
   wherein :
   - X₁ is a glycine or a glutamate residue;
   - X₂ is an aspartate or a tyrosine residue;
   - X₃ is a threonine or a valine or a proline residue;
   - X₄ is a phenylalanine or a proline or a serine residue
   - X₅ is a serine or a glutamate residue;
   - X₆ is a glycine or an asparagine or a serine residue;
   - X₇ is a tyrosine or a glycine or a threonine residue
   - X₈ is a leucine or a tyrosine or an alanine residue; and
   - X₉ is an alanine or a methionine residue;
**(b).**CDR2: Ala-Ile-Asn-Ser-X₁-Arg-His-Thr-Thr-Ser-Tyr-Ala-X2-Ser-Val-Lys-Gly (SEQ ID NO: 13)
   wherein :
   - X₁ is a lysine or a valine residue; and
   - X₂ is an aspartate or an asparagine residue;
      and/or
**(c).** CDR3: Ala-Asp-Ala-Ile-Gly-Leu-Ala-Glu-Tyr-Trp-Ser-Thr-Pro-Thr-Leu-Ser-Ala-Ala-Arg-Tyr-Lys-Tyr (SEQ ID NO: 8).

In a particular embodiment, the VHH of the invention comprises:
- a CDR1 selected from SEQ ID NO: 6 11 or 12 or a sequence having at least 80% sequence identity thereto, preferably at least 90%, 92%, 94%, 95%, 96%, 97%, 98%, 99% or more amino-acid sequence identity thereto; and/or
- a CDR2 selected from SEQ ID NO: 7, 9 or 10 or a sequence having at least 80% sequence identity thereto, preferably at least 90%, 92%, 94%, 95%, 96%, 97%, 98%, 99% or more amino-acid sequence identity thereto; and/or
- a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

In a particular embodiment, the VHH of the invention comprises:
- a CDR1 comprising SEQ ID NO: 6 or a sequence having at least 80% sequence identity thereto, preferably at least 90%, 92%, 94%, 95%, 96%, 97%, 98%, 99% or more amino-acid sequence identity thereto; and
- a CDR2 comprising SEQ ID NO: 7 or a sequence having at least 80% sequence identity thereto, preferably at least 90%, 92%, 94%, 95%, 96%, 97%, 98%, 99% or more amino-acid sequence identity thereto; and
- a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

In a particular embodiment, the VHH of the invention comprises:
- a CDR1 comprising SEQ ID NO: 6 or a sequence having at least 80% sequence identity thereto, preferably at least 90%, 92%, 94%, 95%, 96%, 97%, 98%, 99% or more amino-acid sequence identity thereto; and
- a CDR2 comprising SEQ ID NO: 9 or a sequence having at least 80% sequence identity thereto, preferably at least 90%, 92%, 94%, 95%, 96%, 97%, 98%, 99% or more amino-acid sequence identity thereto; and
- a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

In a particular embodiment, the VHH of the invention comprises:
- a CDR1 comprising SEQ ID NO: 6 or a sequence having at least 80% sequence identity thereto, preferably at least 90%, 92%, 94%, 95%, 96%, 97%, 98%, 99% or more amino-acid sequence identity thereto; and
- a CDR2 comprising SEQ ID NO: 10 or a sequence having at least 80% sequence identity thereto, preferably at least 90%, 92%, 94%, 95%, 96%, 97%, 98%, 99% or more amino-acid sequence identity thereto; and
- a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

In a particular embodiment, the VHH of the invention comprises:
- a CDR1 comprising SEQ ID NO: 11 or a sequence having at least 80% sequence identity thereto, preferably at least 90%, 92%, 94%, 95%, 96%, 97%, 98%, 99% or more amino-acid sequence identity thereto; and
- a CDR2 comprising SEQ ID NO: 9 or a sequence having at least 80% sequence identity thereto, preferably at least 90%, 92%, 94%, 95%, 96%, 97%, 98%, 99% or more amino-acid sequence identity thereto; and
- a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

In a particular embodiment, the VHH of the invention comprises:
- a CDR1 comprising SEQ ID NO: 12 or a sequence having at least 80% sequence identity thereto, preferably at least 90%, 92%, 94%, 95%, 96%, 97%, 98%, 99% or more amino-acid sequence identity thereto; and
- a CDR2 comprising SEQ ID NO: 9 or a sequence having at least 80% sequence identity thereto, preferably at least 90%, 92%, 94%, 95%, 96%, 97%, 98%, 99% or more amino-acid sequence identity thereto; and
- a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

VHH polypeptides of the invention typically comprise four framework regions FR1, FR2, FR3, and FR4, which may be selected e.g., from conventional FR regions of VHH polypeptides. The FR regions may, more preferably, be selected from FR1-FR4 domains having a sequence as defined below:
FR1: Gln-Val-Gln-Leu-Gln-Glu-Ser-Gly-Gly-Gly-X₁-Val-Gln-X₂-Gly-Gly-Ser-Leu-X₃-X₄-X₅-Cys-X₆-Ala-Ser (SEQ ID NO: 15)
wherein :
   - X₁ is a serine or an alanine residue;
   - X₂ is a valine, a proline or an alanine residue;
   - X₃ is an arginine or a lysine residue;
   - X₄ is a valine or a leucine residue
   - X₅ is an alanine or a serine residue; and
   - X₆ is an alanine, a glutamate or a valine residue;
FR2: Trp-Phe-Arg-Gln-Ala-Pro-Gly-Lys-X₁-Arg-Glu-Gly-Val-Ala (SEQ ID NO: 16)
wherein :
   - X₁ is a glycine or a glutamate residue;
FR3: Arg-Phe-Thr-Ile-Ser-Lys-Asp-Asn-Ala-Asp-Asn-X₁-Met-Tyr-Leu-Glu-Met-Asn-X₂-Leu-Lys-Pro-Glu-Asp-Thr-Ala-Ile-Tyr-Tyr-Cys-Ala-Ala (SEQ ID NO: 17)
wherein :
   - X₁ is a isoleucine or a methionine residue; and
   - X₂ is a serine or a glycine residue; and/or
FR4: Trp-Gly-Gln-Gly-Thr-Gln-Val-Thr-Val-Ser-Ser (SEQ ID NO: 18).

In a specific embodiment, the VHH of the invention comprises a sequence selected from anyone of SEQ ID NOs: 1-5 or a sequence having at least 80% sequence identity thereto, preferably at least 90%, 92%, 94%, 95%, 96%, 97%, 98%, 99% or more amino-acid sequence identity thereto. The invention also relates to single heavy chain variable domain immunoglobulin (VHH) which bind the same antigen as a VHH of anyone of SEQ ID NO: 1 to 5.

VHHs can be prepared by standard production, isolation and purification methods or by recombinant or synthesis technology. For example, anti-GFLV VHHs can be generated e.g. by immunization of a camelid animal with purified GFLV particles. The DNA molecule encoding said VHH can be determined or isolated or cloned by methods well-known in the art. VHH having specific sequences as defined above can be produced by artificial synthesis or recombinant DNA technology.

The invention also relates to a nucleic acid molecule comprising a nucleotide sequence encoding a polypeptide or VHH as defined above. The nucleic acid may be DNA or RNA. The nucleic acid may further comprise regulatory domains (e.g., a promoter or terminator sequence). The nucleic acid may be cloned in suitable cloning or expression vectors, such as plasmids, viruses, cosmids, phages, etc.

### Methods of the invention

As discussed above, the present invention relates to VHH-mediated GFLV-resistance in plants. The invention stems from the identification of particularly potent VHH molecules as defined above as well as from the demonstration that VHHs can be expressed in plants and confer high resistance to GFLV.

Therefore, according to a particular embodiment, the invention relates to a method for conferring or increasing resistance to *Grapevine fanleaf virus* (GFLV) to plants by introducing (e.g., delivering or expressing) in said plant or a cell thereof an anti-GFLV VHH, anti-GFLV VHH polypeptide in particular.

In a first embodiment, expression of the VHH is obtained by introduction into a plant or plantlet or a plant cell of a nucleic acid construct comprising a nucleic acid sequence encoding said VHH under control of a promoter enabling the expression of said nucleic acid sequence. Cells of the plant thus obtained comprise one or several copies of the nucleic acid sequence and encode the VHH. Such introduction may be performed by e.g., virus-mediated infection. Such a method can be practiced on preexisting and/or cultivated plant, preferentially perennial plants.

In another embodiment, introduction of the VHH is performed by grafting the plant onto a graft (rootstock or scion) that expresses the VHH. By grafting, the VHH or a nucleic acid encoding the VHH (e.g., an RNA) is transferred from the graft to the plant. Such transfer may also be performed by agrocapsule or microcapsule delivery, e.g. by using agrobody.

In a further embodiment, the VHH is expressed in the plant or plant cell by transgenesis (e.g., by generation of a plant from a cell containing the VHH nucleic acid).

The invention thus provides a method for producing a plant having increased resistance to GFLV, wherein the method comprises introducing into said plant an anti-GFLV VHH.

In a specific embodiment, the method comprises the following steps:
a. introducing into a cell of said plant a nucleic acid construct comprising a nucleic acid sequence encoding a VHH under the control of a promoter enabling the expression of said nucleic acid sequence ;
b. optionally, selecting plant cells of step (a) which express the VHH;
c. regenerating plants from cells of step (a) or (b); and
d. optionally, selecting a plant of (c) with increased resistance to GFLV.

As mentioned above, constitutive VHH production in a plant may be obtained by the generation of plants expressing a VHH transgene under the control of a constitutive promoter. VHH production in the plant may also be transient, e.g., by expressing a VHH transgene under the control of an inducible or regulated or development-stage-specific promoter. Preferred expression is a constitutive expression under control of a constitutive promoter. Such constitutive expression, as illustrated in the examples, leads to an expression of an active VHH in the plant, while the plant is still viable.

In a preferred method, the promoter is a heterologous promoter functional in plant cells. Examples of such promoters include, but are not limited to, constitutive promoters, tissue-specific promoters, development-stage-specific promoters, inducible promoters, viral promoters as well as synthetic or other natural promoters. In a preferred aspect, the promoter is a constitutive or an inducible promoter. Constitutive promoters may include, for example grapevine specific promoter VvUb6-1 and VvUb7-2 promoters (Li et al, 2012), promoters derived from a PR1 gene and a PAL gene, Cauliflower mosaic virus 35S (CaMV) 35S promoter (Odell et al., 1985) and ubiquitin promoter (Christensen et al., 1989) and the like.

As shown in the experimental section, homozygous *Nicotiana benthamiana* plant lines can be fully resistant to a broad range of GFLV isolates whether the virus is inoculated mechanically, by nematode or as an RNA, which is particularly surprising and remarkable.

The invention also relates to a method for inducing resistance to GFLV in a plant, more preferably a cultivated plant, wherein said plant is virally infected to introduce into at least a cell of the plant a nucleic acid construct comprising a nucleic acid sequence encoding a VHH under the control of a promoter enabling the expression of said nucleic acid sequence.

A nucleic acid molecule encoding a VHH or the VHH itself may be introduced into a plant cell by any means of biological or mechanical inoculations, including transfection, transformation, transduction, electroporation, particle bombardment, agroinfection, grafting etc. In a preferred embodiment, a nucleic acid molecule is introduced via agrobacterium transformation using the Ti plasmid as described e.g., by Toki et al. (2006) or via vectors including virus-mediated infection (Dolja and Koonin, 2013).

The invention also relates to method to eradicate GFLV from an infected plant, the method comprising introducing or expressing into cells of the plant an anti-GFLV VHH.

Selection of a plant which expresses a VHH can be made by techniques known per se to the skilled person (e.g., PCR, hybridization, use of a selectable marker gene, protein dosing, western blot etc.).

Plant generation from the modified cells can be obtained using methods known per se to the skilled worker. In particular, it is possible to induce, from callus cultures or other undifferentiated cell biomasses, the formation of shoots and roots. The plantlets thus obtained can be planted out and used for cultivation. Methods for regenerating plants from cells are described, for example, by Fennell et al. (1992); Stöeger et al. (1995); Jahne et al. (1994); Krastanova et al. (1995).

The resulting plants can be bred and hybridized according to techniques known in the art. For perennial plants such as Vitis spp, vegetative propagation is preferred to ensure that the genotype or phenotype is stable and inherited.

Selection of plants having an increased resistance to GFLV can be done by inoculating GFLV to the plant, determining resistance and comparing to a wild type plant. Within the context of this invention, the term "increased" resistance to GFLV means a resistance superior to that of a control plant such as a wild type plant, to which the method of the invention has not been applied. The "increased" resistance also designates for instance a reduced, weakened or prevented manifestation of the disease effects or symptoms provoked by a GFLV, a reduction in virus compared to a wild type plant, or a reduced infection rate upon natural transmission of the virus by a vector, such as nematodes.

The GFLV infection symptoms typically comprise symptoms which directly or indirectly lead to an adverse effect on the Vitis genus plant, the harvest yield, the quality of grapes, its use for feeding, sowing, growing, harvesting, etc. Such symptoms include for example infection and lesion of a plant or of a part thereof (e.g., different tissues, leaves, flowers, fruits, seeds, roots, shoots). Leaf symptoms are commonly: Fanleaf deformation - Leaves are asymmetric with an open petiolar sinus. The main veins are drawn close together and teeth along the margin of the leaf blade are elongated; Yellow mosaic - Leaf blades develop a bright yellow color involving the entire leaf or in irregular patches across the leaf blade; and Vein banding - Bright yellow bands may develop along the major veins, starting in early or midsummer. The canes and foliage could also appear clustered because of stunting. Internodes may be irregularly spaced and shorter on canes hence the name court-noué in French to designate the disease. Canes may develop secondary shoots (breaking of bud dormancy) or split. Sometimes, canes become fasciated and tendrils occasionally develop into lateral shoots. Fanleaf virus can also greatly reduce fruit set (up to 80% in some varieties, under some conditions), etc. Preferably, according to the invention, the disease symptoms or virus content of the plant are reduced by at least 5% or 10% or 15%, more preferably by at least 20% or 30% or 40%, particularly preferably by 50% or 60%, most preferably by 70% or 80% or 90% or more, in comparison with the control plant.

In the most preferred embodiment, the method of the invention is used to confer or increase resistance to GFLV of a plant of Vitis spp, in particular a Vitis rootstock. The invention may be used to confer resistance to various GFLV strains, particularly to GFLV strains recognized by commercial immunodiagnostic methods such as DAS-ELISA. As illustrated below, the plants can show resistance to various GFLV strains such as GFLV-F13, GFLV-GHu, GFLV-TD or other strains.

### VHH expressing plants

The invention further relates to a plant or a plant cell expressing an anti-GFLV VHH. Preferably, the plant or cell is selected from *Vitis vinifera, Vitis labrusca, Vitis riparia, Vitis aestivalis, Vitis rotundifolia, Vitis rupestris Vitis amurensis, Vitis coignetiae, Vitis vulpine* species or any hybrid Vitis thereof. In a particular embodiment, the plant is a cultivated rootstock *Vitis spp* used for grafting. Preferred Vitis genus plant or Vitis genus plant cell of the invention exhibit a resistance to GFLV.

In another embodiment, the invention relates to transgenic plants or plant cells which have been engineered to be more resistant to GFLV by expression of a VHH.

The invention also relates to propagation by vegetative reproduction, vegetative propagation, vegetative multiplication, vegetative cloning or cuttings from the plant having an increased resistance to GFLV.

The invention also relates to a seed of a plant of the invention, as well as to a plant, or a descendent of a plant, grown or otherwise derived from said seed, said plant having an increased resistance to GFLV.

The invention also relates to vegetal material of a plant of the invention, such as roots, leaves, flowers, callus, etc.

### Nucleic acids molecules, expression cassettes and vectors

The present invention also relates to nucleic acid molecules suitable for use in the above methods and/or for constructing plants of the invention. More specifically, the invention relates to any nucleic acid molecule encoding a polypeptide or VHH as defined above.

In a particular embodiment, the invention relates to a recombinant expression cassette comprising a nucleic acid molecule as described above, operably linked to a promoter or other regulatory elements functional in a plant such as terminator fragments, polyadenylation sequences, enhancer sequences, reporter genes and other sequences as appropriate.

"Operably linked" means joined as part of the same nucleic acid molecule, suitably positioned and oriented for transcription to be initiated from the promoter. A gene operably linked to a promoter is "under transcriptional initiation regulation" of the promoter.

The promoters useful in the expression cassettes of the invention include, but are not limited to, constitutive promoters, plant tissue-specific promoters, plant development-stage-specific promoters, inducible promoters, viral promoters as well as synthetic or other natural promoters. In a preferred aspect, the promoter is a constitutive or an inducible promoter, as described above.

The present invention also relates to a recombinant vector comprising a nucleic acid molecule or a recombinant expression cassette as described above. Numerous vectors are available for plant transformation, and the nucleic acid molecules, constructs and expression cassettes of the invention may be used in conjunction with any such vectors. Several plant viruses have been engineered into vectors for the delivery of genetic constructs to plants. Among those virus vectors, three of them, i.e. *Grapevine virus A* (GVA) (Muruganantham et al., 2009), *Grapevine leafroll-associated virus 2* (GLRaV-2) (Kurth et al. 2012) and *Grapevine rupestris stem pitting-associated virus* (GRSPaV) (Meng et al., 2013) that were designed to deliver heterologous proteins or silence host genes in grapevines are particularly relevant to the present invention due to their use in Vitis.

The selection of the suitable vector for uses and methods of the invention will depend upon preferred transformation technique and the target species for transformation.

The present invention also relates to transformed plant cells into which the vectors of the present invention have been inserted and to methods for producing plants having resistance to GFLV using such transformed cells.

Further aspects and advantages of the invention are provided in the following examples, which are given for purposes of illustration and not by way of limitation.

### EXAMPLES

### A- MATERIALS AND METHODS

### Immunization, V_{H}H library construction and screening

GFLV-specific single domain antibodies (Nanobodies (Nbs) or VHH) were generated according to published methods (Ghassabeh et al. 2010). In brief, a camel *(Camelus dromedarius*) was injected 6 times subcutaneously at weekly intervals with 100 µg of purified GFLV strain F13 (Serghini et al. 1990; Ritzenthaler et al. 1991) according to standard immunization protocols. After immunization, total RNA was extracted from isolated peripheral blood lymphocytes and mRNAs reverse transcribed to cDNA. The VHH genes were then amplified with two subsequent nested PCR, cloned into the pHEN4 phagemid vector (Ghahroudi et al. 1997) and transformed into *E. coli* TG1 cells. The resulting VHH library was screened by phage display for GFLV-specific binders in 3 consecutive biopanning rounds against 10 µg of purified GFLV-F13 each. Sequences of GFLV-specific Nanobodies were obtained following the isolation of individual clones from the enriched library by a phage-ELISA approach performed against 100 ng of purified GFLV-F13.

### VHH E. coli expression and purification

Selected GFLV-specific Nanobodies coding sequences were subcloned into the pHEN6c expression vector as a BstEII/PstI fragment adding a N terminal pelB signal sequence for periplasmic targeting and a C terminal His₆-tag for purification. Large-scale production of His₆-tagged VHH constructs was performed by expression in freshly transformed *E*. *coli* WK6 cells grown in Terrific Broth (TB) medium and induced overnight with 1 mM isopropyl-ß-D-thiogalactopyranoside (IPTG) at 28°C (Thys et al. 2010).

An additional C terminal Strep-tag II was added for Nbs use in ELISA. To do so, VHH coding sequence were amplified by PCR using primer pair #152/#197 (Table 1) and amplicons introduced by Gateway cloning into the pDONR/Zeo vector [Invitrogen] which was further recombined into the p0GWA expression vector (Busso et al. 2005). Large-scale production of Strep II tagged VHH constructs was performed by expression in freshly transformed *E*. *coli* BL21 (DE3) cells grown overnight in auto-inducing medium ZYP 50502 (Studier 2005) at 23°C.

Periplasmic VHH were extracted by osmotic shock (Habib et al. 2013) and purified by immobilized metal ion chromatography (IMAC) on a 1 ml Protino Ni-NTA column [Macherey-Nagel] using 500 mM imidazole in running buffer (50 mM Tris, 300 mM NaCl, 5% glycerol, pH 8.0) for elution, followed by size exclusion chromatography (SEC) on a Hiload 16/60 Superdex 75 prep grade column [GE Healthcare Life Science] in PBS (Thys et al. 2010; Conrath et al. 2001). The purity of the eluted proteins was assessed in a Coomassie stained 16% Tris-Tricine SDS PAGE in which Nanobodies appeared as single bands of ∼15 kDa. Final yields of purified Nanobodies were estimated from their 280 nm UV absorbance using theoretical extinction coefficients computed from their respective amino acid contents.

### DAS-ELISA assessment of Nb reactivity against GFLV strains

Plants (4- to 6-leaf stage) were mechanically inoculated with crude saps from *Chenopodium quinoa* infected with GFLV-GHu (Vigne et al. 2008), -F13, -B844 (Legin et al. 1993), -TD (Schellenberger et al. 2011), -CO2 (Vigne et al. 2004), -BuCharT60, - BE 4.11, -BE 5.19 and ArMV-S (Huss et al. 1989). Infected systemic leaves were ground at 14 dpi in extraction buffer (35 mM Na₂HPO₄, 15 mM KH₂PO₄, pH 7.0) in a 1:5 (w/v) ratio. Virus detection was performed on clarified extracts by DAS-ELISA using anti-GFLV or anti-ArMV polyclonals [Bioreba] diluted 1,000-fold in coating buffer (15 mM Na₂CO₃, 35 mM NaHCO₃, pH 9.6) as capture antibody and the StrepII tagged Nb23 at 1 µg/ml in conjugate buffer (10 mM PBS, 0.1% BSA, 0.05% Tween 20, pH 7.4) as detection antibody. For development, streptavidin-alkaline phosphatase [Jackson Immunoresearch] at 50 ng/ml in conjugate buffer was used in conjunction with pNPP (para-nitrophenyl phosphate) [Interchim] at 1 mg/ml in substrate buffer (1 M diethanolamine, pH 9.8). Negative control consisted of non-inoculated healthy plants. Absorbance at 405 nm (A₄₀₅ₙₘ) was recorded after one hour of substrate hydrolysis and samples with mean A₄₀₅ₙₘ values exceeding by a factor of 2.4 those of negative controls were considered positive. Results are presented as mean A₄₀₅ₙₘ values of experimental duplicates ± standard error normalized against maximum assay values.

### VHH: EGFP generation and plant

The Nanobody Nb23 was cloned in frame to the N-terminus of EGFP with a Gly₃SerGly₃ linker sequence into the pEAQ HT DEST3 plant expression vector (Sainsbury et al. 2009). EGFP was first introduced into the pHEN6c Nb23 vector as an EcoRI/BstEII fragment following a PCR amplification using primers pair 429/430 (Table 1). The Nb23:EGFP gene was then re-amplified by PCR using primer pair #153/#150 (Table 1) and introduced by Gateway cloning into the pDONR/Zeo vector [Invitrogen] which was further recombined into the pEAQ HT DEST3 vector. A control consisting of pEAQ HT DEST3 EGFP was included.

The resulting vectors were transferred into *Agrobacterium tumefaciens* GV3101: :pMP90 cells (Koncz and Schell 1986) and used at OD₆₀₀ₙₘ= 0.1 for agro-transformation of *Nicotiana benthamiana* leaves as described (Sparkes et al. 2006). After 3 days, expression of fluorescence was checked and sterilized infiltrated leaf segments were placed in a growth chamber (16h light/8h dark, 25°C) onto shoot induction medium (Murashige and Skoog (MS) medium [M0238, Duchefa], 10 mM NH₄NO₃, 1x MS vitamin solution [M2909, Sigma], 3% (w/v) sucrose, 0.05 µg/ml 1-naphthalene acetic acid (NAA), 2 µg/ml 6-benzyl-aminopurine (BAP), 0.8% (w/v) agar, 150 µg/ml kanamycin , 500 µg/ml carbenicillin, pH 5.8). Callus were subcultured every week onto fresh medium and shoots excised 3 to 4 weeks later and transferred onto rooting medium (1/2 MS medium, 1.5% (w/v) sucrose, 0.5x MS vitamin solution, 0.8% (w/v) agar, 150 µg/ml kanamycin, 500 µg/ml carbenicillin, pH 5.8) until plantlets could be acclimated and established in soil. Regenerated T0 plants were self pollinated through T2 generation and genomic insertion sites of the transgene was determined by tail PCR (Wang et al. 2011; Singer & Burke 2003).

For Nb23: EGFP large-scale production, the whole Nb23: EGFP: His6 coding sequence was cloned into the pET22b expression vector [Novagen] as a NdeI/XhoI fragment subsequent to a PCR amplification with primer pair #397/#399 (Table 1) using pEAQ HT DEST3 Nb23: EGFP as template. Expression was performed as previously described (Habib et al. 2013) in freshly transformed *E. coli* SHuffle T7 Express cells [New England Biolabs] grown in TB medium and induced overnight with 0.1 mM IPTG at 20°C. Pelleted cells resuspended in PBS-NaCl buffer (10 mM PBS, 300 mM NaCl, pH 7.4) were lysed by sonication (80% amplitude for 2 min with 13 mm diameter probe, Vibra-Cell VCX 500 [Sonics]) and purification of cytoplasmic extract carried out as indicated above. The purity of the eluted protein was assessed in a Coomassie stained 12.5% acrylamide Tris-Glycine SDS PAGE in which Nb23: EGFP was detected as a band of ∼43 kDa.

### Quantification of Nb23: EGFP expressed in N. benthamiana plants

Amounts of recombinant proteins expressed in transgenic plants were estimated by fluorometry and immunoblotting analyses. To do so, the 3 youngest apical leaves of 6-7 weeks old *N. benthamiana* plants were homogenized in extraction buffer (200 mM Tris HCl, 300 mM NaCl, 100 mM ascorbic acid, 2.5% polyvinylpolypyrrolidone (PVPP), complete protease inhibitor cocktail [Roche], pH 7.0) in a 1:2 (w/v) ratio and cell debris removed by centrifugation at 20,000 x g for 20 min at 4°C.

Fluorescence intensity was recorded in a FLUOstar Omega plate reader [BMG Labtech] equipped with 485/12 nm excitation and 520/25 nm emission filters, on 100 µl of extracts in a white flat bottom polystyrene plate [Greiner Bio One]. WT *N. benthamiana* extracts were used as blank and purified Nb23: EGFP diluted in extracts from WT plants as fluorescent standard.

Total soluble protein concentrations were determined using the BioRad protein assay following manufacturer's protocol with serial dilutions of bovine serum albumin (BSA) in extraction buffer as standard.

Total soluble proteins were acetone-precipitated, heat-denaturated in protein denaturing buffer, separated through a 12.5% acrylamide Tris-Glycine SDS-PAGE and transferred to a polyvinylidene difluoride (PVDF) membrane using the Trans Blot Turbo transfer system [BioRad]. After incubation in blocking buffer (10 mM PBS, 0,1% Tween-20, 5% skim milk), the membrane was sequentially probed with rabbit anti-GFP IgG [G-1544, Sigma] at 0.1 µg/ml in blocking buffer and a goat anti-rabbit IgG conjugated to horseradish peroxidase [G-21234, Life Technologies] at 0.1 µg/ml in blocking buffer. Immunolabeled proteins were detected by enhanced chemiluminescence (ECL) using the Lumi-Light kit [Roche] and the Fusion FX imaging system [Vilber Lourmat].

### Dynamic Light Scattering (DLS) analyses

Viral particles were purified as described previously (Schellenberger et al. 2011). Mean particle diameters and polydispersity of GFLV F13 alone or complexed to Nb23 or to Nb23: EGFP was estimated by DLS (Lorber et al. 2012) using a Zetasizer NanoS [Malvern]. At least 5 successive measurements were performed for each combination with virus at 0.1 mg/ml in Tris buffer (50 mM Tris, 100 mM NaCl, pH 8.3), Nb23 at 0.1 mg/ml and Nb23:EGFP at 0.9 mg/ml. Scattered intensities were recorded at 20°C and data processed with DTS software (version 6.01).

### Reverse Transcriptase-qPCR (RTqPCR) analyses

The accumulation of GFLV RNA1, Nanobodies/EGFP and P19 transcripts were quantified by RTqPCR relatively to the expression of 3 endogenous genes. Total RNA was isolated at 21 dpi from approximately 17 mg of uninoculated apical leaves ground at a 1:30 (w/v) ratio in TLES-buffer (100 mM Tris, 100 mM LiCl, 10 mM EDTA, 0.1% (w/v) SDS, pH 8.0) followed by a water-saturated phenol and phenol chloroform extraction before precipitation with 2 M LiCl. cDNA was generated according to manufacturers' instructions from 1µg of DNase I treated total RNA using 2.5 µM Oligo(dT)18 primer [Fermentas] and SuperScript III Reverse Transcriptase [Invitrogen]. PCR was performed in triplicates using 0.5 µl of reverse transcription reaction and 2.5 µM gene specific primers (Table 2) in a total volume of 10 µl LightCycler 480 SYBR Green Master I mix [Roche] on a LightCycler 480 system [Roche] with cycling conditions of 5 min denaturation at 95°C followed by 40 cycles at 95°C for 10 s, 60°C for 15 s and 72°C for 15s. The *N. benthamiana* cyclin dependent kinase homolog (GI:849067, *N. tabacum*), elongation factor 1 alpha (GI:37783254) and actin (GI:380505031) genes were used as internal controls due to their stability assessed by GeneNorm (Vandesompele et al. 2002) and NormFinder (Andersen et al. 2004) algorithms. Relative gene expression levels were calculated by means of the linear regression of efficiency method using LinRegPCR version 2013.0 software (Ruijter et al. 2009).

### GFLV challenge assays

Plants were mechanically inoculated with either purified virus, saps of infected *C. quinoa* plants or purified viral RNA. Viral RNA was isolated from purified virus using RNeasy MinElute Cleanup kit [Qiagen] after phenol/chloroform extraction.

Nematode transmission assay was performed by growing plants on soil containing ∼300 viruliferous *Xiphinema index* per plant for 6 weeks.

DAS-ELISAs were performed at 21 dpi on apical uninoculated leaves using in-house anti-GFLV polyclonal or anti-GFLV detection kit [Bioreba] according to standard protocols and manufacturer instructions.

### Confocal Laser Scanning microcopy

Water-mounted leaves disks of 6-7 weeks old *N. benthamiana* plants were imaged using LSM 780 laser scanning confocal mounted on observer Z1 microscope [Zeiss] equipped with a 20x/0.8 Plan-Apochromat objective. Excitation/ Emission wavelengths were 488 nm/505-525 nm and images were processed using Zen 2011 imaging software [Zeiss].

**Table 1 List of primers used for VHH cloning (5'-3' sequences)**

| |
|---|
| #152 GGGGACAAGTTTGTACAAAAAAGCAGGCTCTATGAAATACCTATTGCCTACGG (SEQ ID NO: 19) |
| #197 GGGGACCACTTTGTACAAGAAAGCTGGGTCTTATTTTTCAAATTGAGGATGAGACCAGTG ATGGTGATGGTGGTG (SEQ ID NO: 20) |
| 429 ATCTTAggtcaccGTCTCCTCAGGC*GGCGGTAGCGGCGGCGG*TATGGTGAGCAAGGGCGA (SEQ ID NO: 21) |
| 430 TACTCTgaattcTATTAGTGATGATGATGGTGGTGCTTGTACAGCTCGTCCAT (SEQ ID NO: 22) |
| #153 GGGG**ACAAGTTTGTACAAAAAAGCAGGCT**CTATGCAGGTGCAGCTGCAGG (SEQ ID NO: 23) |
| #150 GGGG**ACCACTTTGTACAAGAAAGCTGGGT**CCTTGTACAGCTCGTCCATG (SEQ ID NO: 24) |
| #397 TCAAATcatatgCAGGTGCAGCTGCAGG (SEQ ID NO: 25) |
| #399 GTGActcgagTTAGTGATGGTGATGGTGATG (SEQ ID NO: 26) |

Gateway recombination sequences are indicated in bold. Strep-tag II sequence is underlined. Linker Gly₃SerGly₃ sequence is in italics. BstEII, EcoRI, NdeI and XhoI restriction sites are indicated in lower case.

**Table 2 List of primers used for qPCR**

| PRIMERS | TARGETS | **5'-3'** SEQUENCES | **SEQUENCE NUMBER** |
|---|---|---|---|
| RNA1-fwd | GFLV-1D | CCCAAAAGTCATCGCAATGCT | SEQ ID NO: 27 |
| RNA1-rev | GFLV-1E | GGATCAGGATATGGAAAGCAC | SEQ ID NO: 28 |
| EGFP-fwd | EGFP | TATATCATGGCCGACAAGCA | SEQ ID NO: 29 |
| EGFP-rev | EGFP | GAACTCCAGCAGGACCATGT | SEQ ID NO: 30 |
| P19-fwd | TBSV-P19 | TGAGATGGCAATTCGGTCTA | SEQ ID NO: 31 |
| P19-rev | TBSV-P19 | TGATACATTACTTTCCACTTCGAT | SEQ ID NO: 32 |
| CDC2-fwd | Cyclin dependent kinase | GTGTAGTGTACAAGGCTCGTGA | SEQ ID NO: 33 |
| CDC2-rev | Cyclin dependent kinase | TAGCTGTGCTTGGTACTCCCTC | SEQ ID NO: 34 |
| EF1a-fwd | Elongation factor 1-alpha | GTACTGTCCCTGTTGGTCGT | SEQ ID NO: 35 |
| EF1a-rev | Elongation factor 1-alpha | GTAGGTCCAAAGGTCACAACCAT | SEQ ID NO: 36 |
| ACT-fwd | actin | AAATTACTGCACTTGCTCCTAGC | SEQ ID NO: 37 |
| ACT-rev | actin | CAATCCAGACACTGTATTTCCTCTC | SEQ ID NO: 38 |

### EXAMPLE 1: GENERATION OF GFLV-SPECIFIC NANOBODIES

In a first step to generate nanobodies specific for GFLV, a camel was immunized with purified GFLV-F13 isolate and a phagemid library was generated, representing the respective VHH (nanobody) repertoire. In a first screening cycle, more than 20 nanobodies were identified as binding to GFLV. In order to further improve the stringency of the nanobodies, three subsequent phage display cycles were performed and five VHH sequences named Nb23, Nb101, Nb126, Nbp71 and Nbp75 were selected, each consisting of 131 residues (Figure 1). The full sequences were determined and the nanobodies were cloned with a C-terminal 6His-tag, expressed in *E. coli* and purified by immobilized metal ion affinity chromatography and size-exclusion chromatography, as illustrated in Figure 2 for Nb23 and Nb 126.

The amino acid sequences of all 5 Nbs are provided in the Sequence listing.
Antigen recognition and specificity of Nb23 towards different GFLV isolates and ArMV-S was tested by DAS-ELISA (Figure 3B). Remarkably, all tested GFLV isolates (GFLV-GHu, -F13, -B844, -TD, -CO2, -BUChardT60, -BE4.11 and -BE5.19) showed significant binding response to Nb23, whereas ArMV-S failed to be recognized. This suggests that Nb23 is GFLV-specific and recognizes a broad spectrum of GFLV isolates but is unable to detect ArMV in ELISA.

To gain further insight in Nb23 recognition, a fusion protein between Nb23 and enhanced green fluorescent protein (EGFP) containing a Gly₃SerGly₃ linker peptide was generated (Nb23: EGFP), expressed in *E coli* and purified to near homogeneity. When tested by dynamic light scattering (DLS) against GFLV-F13, an increase in average hydrodynamic diameter of viral particles from 32 nm to 37.8 and 43.8 nm in the presence of Nb23 and Nb23: GFP, respectively, was observed (Figure 3C). It is concluded that fusion of Nb23 to EGFP does not interfere with the capacity of Nb23 to bind specifically to GFLV *in vitro.*

### EXAMPLE 2: EXPRESSION OF THE Nbs IN PLANT AND GFLV RESISTANCE

pEAQDest3-Nb23:EGFP and pEAQDest3-EGFP vectors were used for plant transformation (Figure 4). A total of 10 EGFP and 25 Nb23: EGFP independent *N. benthamiana* T1 lines were analyzed (Figure 5).

When challenged with GFLV-GHu by mechanical inoculation at 300 ng per inoculation, 94% (165 out of 175) of EGFP plants showed symptoms, whereas 53% (299/568) Nb23:EGFP plants remained asymptomatic (Figure 5). By ELISA, all EGFP plants tested positive (69/69) for GFLV, whereas infection rate of only 57% was observed for Nb23: EGFP plants (190/334). These results show that Nb23-expressing plants have very substantial increased resistance to GFLV. Difference in percentage of infection amongst Nb23: EGFP lines is probably due to differences in genotypes and the fact that T1 lines are segregating.
The clear differences in symptoms and infection rates between EGFP and Nb23 :EGFP lines indicate that expression of Nb23:EGFP in transgenic *N. benthamania* T1 lines leads to resistance to GFLV-GHu.

### EXAMPLE 3: GENERATION OF T2 TRANSGENIC LINES EG11-3, 23EG16-9 AND 23EG38-4.

T1 lines 23EG16 and 23EG38 that displayed improved resistance to GFLV-GHu upon mechanical inoculation and the fully susceptible control line Erg11 (see figure 5), where chosen for the production ofhomozygous lines 23EG16-9, 23EG38-4 and EG11-3. In the homozygous lines 23EG16-9 and 23EG38-4, Nb23: EGFP is found in the cytoplasm and nucleus similarly to the localization of EGFP in line EG11-3 (Figure 6A). When quantified by fluorometry, Nb23: EGFP represents approximately 0.12% and 0.07% of total soluble proteins in lines 23EG16-9, 23EG38-4, respectively, whereas EGFP represents approximately 1.73% of total soluble proteins in line EG11-3 (Figure 6B) this indicates that Nb23: EGFP in lines 23EG16-9, 23EG38-4 is respectively 14 to 25 less abundant than EGFP in line EG11-3. By immunoblotting using anti-GFP antibodies for detection, Nb23: EGFP accumulates as a single band of approximately 43 kDa in plants line 23EG16-9, 23EG38-4 that comigrates with purified Nb23: EGFP (lane 9). EGFP is detected in line EG11-3 to much higher level than Nb23: EGFP, confirming the fluorometry results (Figure 6C, note the different loading for EG11-3 vs 23EG16-9, 23EG38-4). mRNA encoding Nb23:EGFP from lines 23EG16-9, 23EG38-4 and EGFP from line EG11-3 were quantified by RTqPCR using same pair of primers. Average level of accumulation was 144AU and 106AU in lines 23EG16-9, 23EG38-4, respectively and 212AU in line EG11-3. (Figure 6D). mRNA encoding Nb23:EGFP from lines 23EG16-9, 23EG38-4 is therefore approximately 1.5 to 2.0 less abundant than mRNA encoding EGFP in line EG11-3. The difference in accumulation of mRNA encoding EGFP vs Nb23: EGFP is about 10 time less important than those of the proteins, suggesting that EGFP and Nb23: EGFP are regulated post-transcriptionally in a different manner. mRNA encoding the suppressor of silencing P19 from lines 23EG16-9, 23EG38-4 and EG11-3 was also quantified by RTqPCR. In these lines, mRNA encoding P19 was detected at very low levels and a maximum 2.2 fold difference in accumulation was seen between the different lines with highest amount of P19 mRNA found in line 23EG16-9 and lowest amounts in line EG11-3. No correlation between P19 mRNA and Nb23: EGFP mRNA or EGFP mRNA accumulation could be observed. This suggests that Nb23: EGFP and EGFP expression is likely P19 independent.

### EXAMPLE 4: RESISTANCE OF T2 LINES 23EG16-9, 23EG38-4, EG11-3 T2 LINES TO GFLV-GHu AND ARMV-S.

Inoculations were performed with 300 ng of purified GFLV-GHu. All WT and EGFP lines showed mosaic symptoms on systemic leaves at 7 days post-inoculation with GFLV-GHu. In contrast, T2 lines 23EG16-9 and 23EG38-4 remained generally asymptomatic (Figure 7) and thus showed strong resistance.

To confirm that absence of symptoms is correlated to the absence virus, T2 lines 23EG16-9 and 23EG38-4 were analyzed by DAS-ELISA (figure 8A, B) and RT-qPCR (Figure 8 C, D). All WT and EGFP lines revealed positive by ELISA, whereas GFLV remained undetectable in the 20 plants from lines 23EG16-9 and in 18 (out of 19) plants from line 23EG38-4. When challenged with ArMV-S, all plants from lines EG11, 23EG16-9, 23EG38-4 were infected similarly to WT plants. These results indicate that lines 23EG16-9 and 23EG38-4 are resistant to GFLV-GHu but not to ArMV-S upon mechanical inoculation with 300 ng of virus. The resistance is associated specifically to Nb23 since EGFP plants are not resistant to GFLV.
Since RTqPCR is considered to be generally more sensitive than ELISA for detection, the same plants were also analyzed by RTqPCR for the presence of GFLV-RNA1 and mRNA encoding EGFP, Nb23: EFGP and P19 (Figure 8C). Amongst lines 23EG16-9 and 23EG38-4 only plant 23EG38-4#17 tested positive for GFLV RNA1 confirming previous ELISA results. Also, accumulation of mRNA encoding EGFP, Nb23: EFGP and P19 were similar to those observed in non-inoculated plants (compare Fig 8C to 6D). Individual RTqPCR analysis of plants from line 23EG38-4 confirmed that GFLV RNA1 is detected only in plant 23EG38-4#17 (Figure 8C, D). This plant was indistinguishable from the other plants of the same line in amounts of Nb23: EFGP or P19 transcripts detected (Figure 8D). This indicates that infection of this plant cannot be attributed to the absence of Nb23: EFGP or to abnormally high amount of P 19 suppressor of silencing that could theoretically promote infection.

### EXAMPLE 5: EVALUATION OF THE RESISTANCE OF T2 LINES 23EG16-9 AND 23EG38-4 TO INFECTION BY VARIOUS GFLV ISOLATES.

EG11-3, 23EG16-9 and 23EG38-4 T2 plants were mechanically inoculated with saps from *C*. *quinoa* plants infected with GFLV isolates -GHu, -F13, -B844, -TD, -CO2,-BUChardT60, -BE4.11 and -BE5.19 and apical non-inoculated leaves analyzed by DAS-ELISA at 21 dpi (Figure 9).

All EG11-3 plants became infected (108/108 plants) with GFLV: 0% resistance.

In sharp contrast, no plant from line 23EG16-9 revealed ELISA positive (100% resistance) and only 3 plants out of 108 (> 97% resistance) from line 23EG38-4 were infected with isolates GFLV-TD, GFLV-CO2 and GFLV-BE4.11. This indicates that Nb23 expression in transgenic plants is able to confer broad range resistance to GFLV isolates upon mechanical inoculation.

### EXAMPLE 6: EVALUATION OF THE RESISTANCE OF T2 LINES 23EG16-9 AND 23EG38-4 TO INFECTION BY VIRAL RNA.

EG11-3, 23EG16-9 and 23EG38-4 plants were mechanically inoculated with viral RNA of GFLV-GHu (360 ng per inoculum) and apical non-inoculated leaves analyzed by DAS-ELISA at 21 dpi (Figure 10).

As with purified virus, 100% of the EG11-3 plants became infected (10/10 plants) with GFLV: 0% resistance.

In sharp contrast, no plant from line 23EG16-9 revealed ELISA positive (100% resistance) and only 1 plant out 10 from line 23EG38-4 was infected (>90% resistance). This indicates that plants 23EG16-9 and 23EG38-4 are resistant to GFLV infection via viral RNA and that Nb23 is able to confer resistance to GFLV viral RNA.

### EXAMPLE 7: EVALUATION OF THE RESISTANCE OF T2 LINES 23EG16-9 AND 23EG38-4 TO GFLV AT HIGH PRESSURE OF INOCULUM.

EG11-3, 23EG16-9 and 23EG38-4 plants were mechanically inoculated with GFLV-GHu at high dose of inoculum (3µg instead of 300 ng per inoculum) and apical non-inoculated leaves analyzed for symptoms at 7, 14 and 21 dpi and by DAS-ELISA at 21 dpi (Figure 11).

All EG11-3 plants displayed symptoms at 7dpi (10/10 plants, 0% resistance) that persisted at 14 dpi but vanished by day 21, probably due to recovery. With line 23EG16-9 and 23EG38-4, no symptoms were observed at 7 dpi (100% resistance), and by dpi 14 and 21, 90% and 70 % of the plants remained GFLV-GHu symptom-free, respectively (Figure 11A). ELISA confirmed results from symptoms assessments except for line 23EG16-9 in which 40% of the plants tested GFLV-positive (Figure 11B). Accordingly, a majority of plants from line 23EG16-9 and 23EG38-4 remained totally resistant to high pressure of inoculum and, when infected, infection was delayed compared to control plants. This indicates that Nb23 is able to confer resistance to GFLV at high pressure of inoculum.

### EXAMPLE 8: EVALUATION OF THE RESISTANCE OF T2 LINES 23EG16-9 AND 23EG38-4 TO GFLV TRANSMISSION BY X. index.

To assess the resistance of EG11-3, 23EG16-9, 23EG38-4 and WT plants to infection via nematode, plants were grown in contaminated soil containing viruliferous *X index.* As negative controls, plants of each genotype maintained in contact with aviruliferous X. *index* were included (NC) (Figure 12). Infection with GFLV was assessed by DAS-ELISA on roots 6 weeks post-contact (Figure 12A) or in leaves 16 weeks post-contact with nematodes (Figure 12B).

GFLV was detected in all roots and in nearly all leaves from EG 11-3 and WT plants demonstrating the efficiency of GFLV transmission by nematodes and the susceptibility to GFLV of EG11-3 and WT plants.

In contrast, GFLV virus remained below detectable levels in roots from plants 23EG16-9 and leaves from plants 23EG16-9 and 23EG38-4. The virus was only detected in roots from 3 23EG38-4 plants, although at lower A₄₀₅ₙₘ values than susceptible controls. This shows that Nb23 confers resistance to GFLV upon transmission of the virus by its natural vector, *X. index.* 23EG16-9 line that expressed slightly higher levels of Nb23: EGFP displayed 100% resistance to GFLV, while line 23EG38-4 displayed slightly lower resistance properties.

These results clearly illustrate the efficacy and remarkable efficiency of anti-GFLV nanobodies to confer resistance in plants.

### REFERENCES

Andersen, C.L., Jensen, J.L. & Ørntoft, T.F. (2004). Normalization of Real-Time Quantitative Reverse Transcription-PCR Data: A Model-Based Variance Estimation Approach to Identify Genes Suited for Normalization, Applied to Bladder and Colon Cancer Data Sets. Cancer research, 64(15), pp. 5245-5250.
Andret-Link, P., Laporte, C., Valat, L., Laval, V., Ritzenthaler, C., Demangeat, G., Vigne, E., Pfeiffer, P., Stussi-Garaud, C., Fuchs, M. (2004a). Grapevine fanleaf virus: Still a major threat to the grapevine industry. Journal of Plant Pathology, 86, pp. 183-195.
Andret-Link, P., Schmitt-Keichinger, C., Demangeat, G., Komar, V. and Fuchs, M. (2004b). The specific transmission of Grapevine fanleaf virus by its nematode vector Xiphinema index is solely determined by the viral coat protein. Virology, 320, pp. 12-22.
Boonrod, K., Galetzka, D., Nagy, P.D., Conrad, U. and Krczal, G. (2004) Single-chain antibodies against a plant viral RNA-dependent RNA polymerase confer virus resistance. Nature Biotechnology, 22, pp. 856-862.
Busso, D., Delagoutte-Busso, B. and Moras, D. (2005). Construction of a set Gateway-based destination vectors for high-throughput cloning and expression screening in Escherichia coli. Analytical Biochemistry, 343(2), pp. 313-321.
Conrath, K.E., Lauwereys, M., Galleni M., Matagne, A., Frère, J-M., Kinne, J., Wyns L. and Muyldermans S. (2001). Beta-lactamase inhibitors derived from single-domain antibody fragments elicited in the camelidae. Antimicrobial Agents and Chemotherapy, 45(10), pp. 2807-2812.
Christensen, A. H. and Quail, H.P. (1989). Sequence analysis and transcriptional regulation by heat shock of polyubiquitin transcripts from maize. Plant Molecular Biology, 12, pp. 619-632,
Demangeat, G., Voisini, R., Minot, J.C., Bosselut, N., Fuchs, M. and Esmenjaud, D. (2005). Survival of Xiphinema index in vineyard soil and retentions of grapevine fanleaf virus over extended time in the absence of host plants. Phytopathology, 95, pp. 1151-1156.
Dolja, V.V. and Koonin, E.V. (2013). The closterovirus-derived gene expression and RNA interference vectors as tools for research and plant biotechnology. Frontiers in Microbiology, doi: 10.3389/fmicb.2013.00083.
Esmenjaud, D. and Bouquet, A. (2009). Selection and application of resistant germplasm for grapevine nematodes management. In: Integrated Management of Fruit Crops and Forest Nematodes Vol.4 A. Ciancio and K. G. Mukerji (eds.), pp. 195-214. Springer Science, New York.
Fennell, A. and Hauptmann, R. (1992). Electroporation and PEG delivery of DNA into maize microspores. Plant Cell Reports, 11, pp. 567-570.
Fuchs, M. and Gonsalves, D. (2007). Safety of virus-resistant transgenic plants two decades after their introduction: lessons from realistic field risk assessment studies. Annual Review of Phytopathology, 45, pp. 173-202.
French, S. and Robson, B. (1983). What is a conservative substitution? Journal of Molecular Evolution, 19(2), pp. 171-175.
Gargouri-Bouzid, R., Jaouna, L., Rouis, S., Saidi, M.N., Bouaziz, D. and Ellouz, R. (2006). PVY-resistant transgenic potato plants expressing an anti-NIa protein scFv antibody. Molecular Biotechnology, 33, pp. 133-140.
Ghahroudi, M., Desmyter, A., Wyns, L., Hamers, R., S. Muyldermans, S. (1997). Selection and identification of single domain antibody fragments from camel heavy-chain antibodies. FEBS letters, 414(3), pp. 521-526.
Ghassabeh, G.H., Saerens, D. and Muyldermans, S. (2010). Isolation of Antigen-Specific Nanobodies. Antibody Engineering. Springer-Verlag. Vol. 2. Berlin Heidelberg pp. 251-266, doi 10.1007/978-3-642-01147-4.
Habib, I., Smolarek, D., Hattab, C., Grodecka, M., Hassanzadeh-Ghassabeh, G., Muyldermans, S., Sagan, S., Gutiérrez, C., Laperche, S., Le-Van-Kim, C., Aronovicz, Y.C., Wasniowska, K., Gangnard, S. and Olivier B. (2013). VHH (nanobody) directed against human glycophorin A: A tool for autologous red cell agglutination assays. Analytical Biochemistry, 438(1), pp. 82-89.
Hamers-Casterman, C., Atarhouch, T., Muyldermans, S., Robinson, G., Hamers, C., Songa, E.B., Bendahman, N., Hamers, R. (1993). Naturally occurring antibodies devoid of light chains. Nature 363, pp. 446-448.
Huss, B., Walter, B. and Fuchs, M. (1989). Cross-protection between arabis mosaic virus and grapevine fanleaf virus isolates in Chenopodium quinoa. Annals of Applied Biology, 114(1), pp. 45-60.
Koncz, C. and Schell, J. (1986). The promoter of TL-DNA gene 5 controls the tissue-specific expression of chimaeric genes carried by a novel type of Agrobacterium binary vector. Molecular and General Genetics, 204(3), pp. 383-396.
Krastanova, S., Perrin, M., Barbier, P., Demangeat, G., Cornuet, P., Bardonnet, N., Otten, L., Pinck L. and Walter B. (1995). Transformation of grapevine rootstocks with the coat protein gene of grapevine fanleaf nepovirus. Plant Cell Reports, 14, pp. 550-554.
Kurth, E.G., Peremyslov, V.V., Prokhnevsky, A.I., Kasschau, K.D., Miller, M., Carrington, J.C. and Dolja, V.V. (2012). Virus-derived gene expression and RNA interference vector for grapevine. Journal of Virology, 86, pp. 6002-6009.
Jahne, A., Becker, D., Brettschneider, R. and Lorz, H. (1994). Regeneration oftransgenic, microspore-derived, fertile barley. Theoretical and Applied Genetics, 89(4), pp. 525-533.
Laimer, M., Lemaire, O., Herrbach, E., Goldschmidt, V., Minafra, A., Bianco, P; and. Wetzel T. (2009). Resistance to viruses, phytoplasmas and their vectors in the grapevine in Europe: A review. Journal of Plant Pathology, 91 (1), pp. 7-23.
Legin, R., Bass, P., Etienne, L. and Fuchs, M. (1993). Selection of mild virus strains of fanleaf degeneration by comparative field performance of infected grapevines. Vitis, 32(2) pp. 103-110.
Li, Z.T., Kim, K-H., Jasinski, J.R., Matthew, R., Creech, M.R. and Gray D.J. (2012). Large-scale characterization of promoters from grapevine (Vitis spp.) using quantitative anthocyanin and GUS assay systems. Plant Science, 196, pp. 132-142.
Lorber, B., Fischer, F., Bailly, M., Roy, H. and Kern, D. (2012). Protein analysis by dynamic light scattering: methods and techniques for students. Biochemistry and Molecular Biology Education, 40(6), pp. 372-382.
Martelli G.P. and Boudon-Padieu E. (2006). Directory of infectious diseases of grapevines and viruses and virus-like diseases of the grapevine: Bibliographic report of 1998-2004 in Options méditerranéennes, SERIE B. Studies and Research Number N 55.
Meng, B., Venkataraman, S., Wang, W., Dayan-Glick, C., Li, C. and Mawassi, M. (2013). Construction and biological activities of the first infectious cDNA clones of the genus Foveavirus. Virology, 435, pp. 453-462.
Muruganantham, M., Moskovitz, Y., Haviv, S., Horesh, T., Fenigstein, A., du Preez, J., Stephan, D., Burger J.T. and Mawassi M. (2009). Grapevine virus A-mediated gene silencing in Nicotiana benthamiana and Vitis vinifera. Journal of Virological Methods, 155, pp. 167-174.
Nölke, G., Fischer, R. and Schillberg, S. (2004). Antibody-based pathogen resistance in plants. Journal of Plant Pathology, 86, pp. 5-17.
Nölke, G., Cobanov, P., Uhde-Holzem, K., Reustle, G., Fischer, R. and Schillberg, S. (2009). Grapevine fanleaf virus (GFLV)-specific antibodies confer GFLV and Arabis mosaic virus (ArMV) resistance in Nicotiana benthamiana. Molecular Plant Pathology, 10, pp. 41-49.
Odell, J.T., Nagy F., and Chua, N-H. (1985). Identification of DNA sequences required for activity of the cauliflower mosaic virus 35S promoter. Nature, 313, pp. 810-812.
Oliver, J. E. and Fuchs, M. (2011). Tolerance and resistance to viruses and their vectors in Vitis sp.: a virologist's perspective of the literature. American Journal of Enology and Viticulture, 62(4), pp. 438-451.
Prins, M., Lohuis, D., Schots, A. and Goldbach, R. (2005) Phage displayselected single-chain antibodies confer high levels of resistance against Tomato spotted wilt virus. Journal of General Virology, 86, pp. 2107-2113.
Ritzenthaler, C., Viry, M., Pinck, M., Margis, R., Fuchs, M. and Pinck, L. (1991). Complete nucleotide sequence and genetic organization of grapevine fanleaf nepovirus RNA1. Journal of General Virology, 72(10), pp. 2357-2365.
Ruijter, J. M., Ramakers, C., Hoogaars, W. M. H., Karlen, Y., Bakker, O., van den Hoff, M. J. B., and Moorman A. F. M. (2009). Amplification efficiency: linking baseline and bias in the analysis of quantitative PCR data. Nucleic Acids Research, 37(6), pp.e45. doi:10.1093/nar/gkp045
Sainsbury, F., Thuenemann, E.C. and Lomonossoff, G.P. (2009). pEAQ: versatile expression vectors for easy and quick transient expression of heterologous proteins in plants. Plant Biotechnology Journal, 7(7), pp. 682-693.
Safarnejad, M., Jouzani, G., Tabatabaie, M., Twyman, R. and Schillberg, S. (2011). Antibody-mediated resistance against plant pathogens. Biotechnology Advances, 29, pp. 961-971.
Sanfaçon, H., Wellink, J., Le Gall, O., Karasev, A., van der Vlugt, R. and Wetzel, T. (2009) Secoviridae: a proposed family of plant viruses within the order Picornavirales that combines the families Sequiviridae and Comoviridae, the unassigned genera Cheravirus and Sadwavirus, and the proposed genus Torradovirus. Archives of Virology, 154, pp. 899-907.
Schellenberger P., Andret-Link P., Schmitt-Keichinger C., Bergdoll M., Marmonier A., Vigne E., Lemaire O., Fuchs M., Demangeat G. and Ritzenthaler C. (2010). A stretch of 11 amino acids in the βB-βC loop of the coat protein of Grapevine fanleaf virus is essential for transmission by Xiphinema index. Journal of Virology, 84, pp. 7924-7933.
Schellenberger, P., Lorber, B., Sauter, C., Bron, P., Trapani, S., Bergdoll, M., Marmonier, A., Lemaire, O., Demangeat, G., and Ritzenthaler, C. (2011). Structural insights into the molecular mechanisms governing Grapevine fanleaf nepovirus transmission by nematodes. PloS Pathogens, 7(5), pp. e1002034.
Serghini, M.A., Fuchs, M., Pinck, M., Reinbolt, J., Walter, B. and Pinck, L. (1990). RNA2 of grapevine fanleaf virus: sequence analysis and coat protein cistron location. Journal of general virology, 71 (7), pp. 1433-1441.
Singer, T. & Burke, E. (2003). High-throughput TAIL-PCR as a tool to identify DNA flanking insertions. Methods in Molecular Biology (Clifton, N.J.), 236, pp. 241-272.
Sparkes I.A., Runions, J., Kearns A., and Hawes C. (2006). Rapid, transient expression of fluorescent fusion proteins in tobacco plants and generation of stably transformed plants. Nature protocols, 1(4), pp. 2019-2025.
Studier, F.W. (2005). Protein production by auto-induction in high-density shaking cultures. Protein Expression and Purification, 41(1), pp. 207-234.
Stöger, E., Fink, C., Pfosser, M. and Heberle-Bors, E. (1995) Plant transformation by particle bombardment of embryogenic pollen. Plant Cell Reports, 14, pp. 273-278.
Thys, B., Schotte, L., Muyldermans, S., Wernery, U., Hassanzadeh-Ghassabeh, G. and Rombaut, B. (2010). In vitro antiviral activity of single domain antibody fragments against poliovirus. Antiviral Research, 87(2), pp. 257-264.
Toki, S., Hara, N., Ono, K., Onodera, H., Tagiri, A., Oka, S. and Tanaka, H. (2006). Early infection of scutellum tissue with Agrobacterium allows high-speed transformation of rice. Plant Journal, 47(6), pp. 969-976.
Vandesompele, J., De Preter, K., Pattyn, F., Poppe, B., Van Roy, N., De Paepe, A. and Speleman, F. (2002). Accurate normalization of real-time quantitative RT-PCR data by geometric averaging of multiple internal control genes. Genome Biology, 3(7), pp. research0034-research0034.11.
Vigne, E., Bergdoll, M., Guyader, S. and Fuchs, M. (2004). Population structure and genetic diversity within Grapevine fanleaf virus isolates from a naturally infected vineyard: evidence for mixed infection and recombination. Journal of General Virology, 85(8), pp. 2435-2445.
Vigne, E., Marmonier, A. and Fuchs, M., (2008). Multiple interspecies recombination events within RNA2 of Grapevine fanleaf virus and Arabis mosaic virus. Archives of Virology, 153(9), pp. 1771-1776.
Villani, M.E., Roggero, P., Bitti, O., Benvenuto, E. and Franconi, R. (2005). Immunomodulation of cucumber mosaic virus infection by intrabodies selected in vitro from a stable single-framework phage display library. Plant Molecular Biology, 58, pp. 305-316.
Wang, Z., Ye, S., Li, J., Zheng, B., Bao, M. and Ning, G. (2011). Fusion primer and nested integrated PCR (FPNI-PCR): a new high-efficiency strategy for rapid chromosome walking or flanking sequence cloning. BMC Biotechnology, 11(1), pp. 109.

## Claims

1. A method for conferring or increasing resistance to *Grapevine fanleaf virus* (GFLV) to a plant, comprising delivering or expressing in said plant a single heavy chain variable domain immunoglobulin (VHH) polypeptide directed against at least one GFLV antigen.

2. The method of claim 1, wherein the VHH is expressed in said plant following genetic transformation of the plant or a cell thereof with a nucleic acid construct encoding said VHH.

3. The method of claim 1 or 2, wherein the nucleic acid construct encoding the VHH is introduced in a cultivated plant by virus-mediated infection.

4. The method of claim 2, wherein the plant is generated from a plant cell comprising a nucleic acid construct encoding the VHH.

5. The method of claim 1, wherein the VHH or a nucleic acid encoding said VHH is delivered to said plant or a cell thereof by grafting, by agrocapsule or microcapsule delivery.

6. A method for eliminating GFLV from an infected plant, the method comprising introducing into cells of said plant an anti-GFLV VHH or a nucleic acid encoding an anti-GFLV VHH.

7. The use of a VHH directed against a GFLV antigen for inducing or increasing resistance to GFLV in a plant.

8. The method according to anyone of claims 1 to 6, or the use according to claim 7, wherein said VHH binds the same antigen as a VHH of anyone of SEQ ID NO: 1 to 5.

9. The method according to anyone of claims 1 to 6, or the use according to claim 7, wherein said VHH comprises:
. a CDR1 selected from SEQ ID NO: 6, 11 or 12 or a sequence having at least 80% sequence identity thereto, preferably at least 90%, 92%, 94%, 95%, 96%, 97%, 98%, 99% or more amino-acid sequence identity thereto; and/or
. a CDR2 selected from SEQ ID NO: 7, 9 or 10 or a sequence having at least 80% sequence identity thereto, preferably at least 90%, 92%, 94%, 95%, 96%, 97%, 98%, 99% or more amino-acid sequence identity thereto; and/or
. a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

10. The method or use of anyone of the preceding claims, wherein said plant is a plant of the Vitis genus, preferably selected from *Vitis vinifera, Vitis vinifera, Vitis, labrusca, Vitis riparia, Vitis aestivalis, Vitis rotundifolia, Vitis rupestris Vitis, amurensis, Vitis coignetiae, Vitis vulpine* or any hybrid thereof.

11. A single heavy chain variable domain immunoglobulin (VHH), wherein said VHH binds the same antigen as a VHH of anyone of SEQ ID NO: 1 to 5.

12. A VHH, which comprises:
. a CDR1 selected from SEQ ID NO: 6, 11 or 12 or a sequence having at least 80% sequence identity thereto, preferably at least 90%, 92%, 94%, 95%, 96%, 97%, 98%, 99% or more amino-acid sequence identity thereto; and/or
. a CDR2 selected from SEQ ID NO: 7, 9 or 10 or a sequence having at least 80% sequence identity thereto, preferably at least 90%, 92%, 94%, 95%, 96%, 97%, 98%, 99% or more amino-acid sequence identity thereto; and/or
. a CDR3 comprising SEQ ID NO: 8 or a sequence having at least 80% sequence identity thereto, preferably at least 90%, 92%, 94%, 95%, 96%, 97%, 98%, 99% or more amino-acid sequence identity thereto.

13. A nucleic acid construct comprising a nucleic acid sequence encoding a VHH of claim 11 or 12.

14. A recombinant vector comprising a nucleic acid construct of claim 13.

15. A cell or plant comprising a VHH of claim 11 or 12, a nucleic acid of claim 13 or a recombinant vector of claim 14.


SEQUENCE LISTING

<110> Institut National de la Recherche Agronomique
      Centre National de la Recherche Scientifique
      VRIJE UNIVERSITEIT BRUSSEL
      Vlaams Instituut voor Biotechnologie VZW

<120> RESISTANCE TO GRAPEVINE FANLEAF VIRUS

<130> B1767EP00

<160> 38

<170> PatentIn version 3.5

<210> 1
<211> 131
<212> PRT
<213> artificial sequence

<220>
<223> Nb126

<400> 1

Gln Val Gln Leu Gln Glu Ser Gly Gly Gly Ser Val Gln Val Gly Gly
1               5                   10                  15

Ser Leu Arg Leu Ala Cys Ala Ala Ser Gly Asp Thr Phe Ser Gly Tyr
            20                  25                  30

Leu Ala Ala Trp Phe Arg Gln Ala Pro Gly Lys Glu Arg Glu Gly Val
        35                  40                  45

Ala Ala Ile Asn Ser Val Arg His Thr Thr Ser Tyr Ala Asn Ser Val
    50                  55                  60

Lys Gly Arg Phe Thr Ile Ser Lys Asp Asn Ala Asp Asn Met Met Tyr
65                  70                  75                  80

Leu Glu Met Asn Ser Leu Lys Pro Glu Asp Thr Ala Ile Tyr Tyr Cys
                85                  90                  95

Ala Ala Ala Asp Ala Ile Gly Leu Ala Glu Tyr Trp Ser Thr Pro Thr
            100                 105                 110

Leu Ser Ala Ala Arg Tyr Lys Tyr Trp Gly Gln Gly Thr Gln Val Thr
        115                 120                 125

Val Ser Ser
    130

<210> 2
<211> 131
<212> PRT
<213> artificial sequence

<220>
<223>  Nb101

<400>  2

Gln Val Gln Leu Gln Glu Ser Gly Gly Gly Ser Val Gln Val Gly Gly
1               5                   10                  15

Ser Leu Arg Leu Ala Cys Ala Ala Ser Gly Asp Thr Phe Ser Gly Tyr
            20                  25                  30

Leu Ala Ala Trp Phe Arg Gln Ala Pro Gly Lys Glu Arg Glu Gly Val
        35                  40                  45

Ala Ala Ile Asn Ser Val Arg His Thr Thr Ser Tyr Ala Asp Ser Val
    50                  55                  60

Lys Gly Arg Phe Thr Ile Ser Lys Asp Asn Ala Asp Asn Met Met Tyr
65                  70                  75                  80

Leu Glu Met Asn Gly Leu Lys Pro Glu Asp Thr Ala Ile Tyr Tyr Cys
                85                  90                  95

Ala Ala Ala Asp Ala Ile Gly Leu Ala Glu Tyr Trp Ser Thr Pro Thr
            100                 105                 110

Leu Ser Ala Ala Arg Tyr Lys Tyr Trp Gly Gln Gly Thr Gln Val Thr
        115                 120                 125

Val Ser Ser
    130

<210>  3
<211>  131
<212>  PRT
<213>  artificial sequence

<220>
<223>  Nb23

<400>  3

Gln Val Gln Leu Gln Glu Ser Gly Gly Gly Ser Val Gln Val Gly Gly
1               5                   10                  15

Ser Leu Arg Val Ala Cys Ala Ala Ser Gly Asp Thr Phe Ser Gly Tyr
            20                  25                  30

Leu Ala Ala Trp Phe Arg Gln Ala Pro Gly Lys Gly Arg Glu Gly Val
        35                  40                  45

Ala Ala Ile Asn Ser Lys Arg His Thr Thr Ser Tyr Ala Asp Ser Val

50                  55                  60

Lys Gly Arg Phe Thr Ile Ser Lys Asp Asn Ala Asp Asn Ile Met Tyr
65                  70                  75                  80

Leu Glu Met Asn Ser Leu Lys Pro Glu Asp Thr Ala Ile Tyr Tyr Cys
                85                  90                  95

Ala Ala Ala Asp Ala Ile Gly Leu Ala Glu Tyr Trp Ser Thr Pro Thr
            100                 105                 110

Leu Ser Ala Ala Arg Tyr Lys Tyr Trp Gly Gln Gly Thr Gln Val Thr
        115                 120                 125

Val Ser Ser
    130

<210>  4
<211>  131
<212>  PRT
<213>  artificial sequence

<220>
<223>  Nbp75

<400>  4

Gln Val Gln Leu Gln Glu Ser Gly Gly Gly Ser Val Gln Ala Gly Gly
1               5                   10                  15

Ser Leu Arg Leu Ser Cys Val Ala Ser Glu Tyr Pro Ser Ser Ser Thr
            20                  25                  30

Ala Met Ala Trp Phe Arg Gln Ala Pro Gly Lys Glu Arg Glu Gly Val
        35                  40                  45

Ala Ala Ile Asn Ser Val Arg His Thr Thr Ser Tyr Ala Asp Ser Val
    50                  55                  60

Lys Gly Arg Phe Thr Ile Ser Lys Asp Asn Ala Asp Asn Met Met Tyr
65                  70                  75                  80

Leu Glu Met Asn Ser Leu Lys Pro Glu Asp Thr Ala Ile Tyr Tyr Cys
                85                  90                  95

Ala Ala Ala Asp Ala Ile Gly Leu Ala Glu Tyr Trp Ser Thr Pro Thr
            100                 105                 110

Leu Ser Ala Ala Arg Tyr Lys Tyr Trp Gly Gln Gly Thr Gln Val Thr
        115                 120                 125

Val Ser Ser
    130

<210>  5
<211>  131
<212>  PRT
<213>  artificial sequence

<220>
<223>  Nbp71

<400>  5

Gln Val Gln Leu Gln Glu Ser Gly Gly Gly Ala Val Gln Pro Gly Gly
1               5                   10                  15

Ser Leu Lys Leu Ser Cys Glu Ala Ser Gly Asp Val Pro Glu Asn Gly
            20                  25                  30

Tyr Met Ala Trp Phe Arg Gln Ala Pro Gly Lys Glu Arg Glu Gly Val
        35                  40                  45

Ala Ala Ile Asn Ser Val Arg His Thr Thr Ser Tyr Ala Asp Ser Val
    50                  55                  60

Lys Gly Arg Phe Thr Ile Ser Lys Asp Asn Ala Asp Asn Met Met Tyr
65                  70                  75                  80

Leu Glu Met Asn Ser Leu Lys Pro Glu Asp Thr Ala Ile Tyr Tyr Cys
                85                  90                  95

Ala Ala Ala Asp Ala Ile Gly Leu Ala Glu Tyr Trp Ser Thr Pro Thr
            100                 105                 110

Leu Ser Ala Ala Arg Tyr Lys Tyr Trp Gly Gln Gly Thr Gln Val Thr
        115                 120                 125

Val Ser Ser
    130

<210>  6
<211>  10
<212>  PRT
<213>  artificial sequence

<220>
<223>  Nb126 CDR1

<400>  6

Gly Asp Thr Phe Ser Gly Tyr Leu Ala Ala
1               5                   10

<210>  7

<211>  17
<212>  PRT
<213>  artificial sequence

<220>
<223>  Nb126 CDR2

<400>  7

Ala Ile Asn Ser Val Arg His Thr Thr Ser Tyr Ala Asn Ser Val Lys
1               5                   10                  15

Gly

<210>  8
<211>  22
<212>  PRT
<213>  artificial sequence

<220>
<223>  CDR3

<400>  8

Ala Asp Ala Ile Gly Leu Ala Glu Tyr Trp Ser Thr Pro Thr Leu Ser
1               5                   10                  15

Ala Ala Arg Tyr Lys Tyr
            20

<210>  9
<211>  17
<212>  PRT
<213>  Artificial sequence

<220>
<223>  Nb101 CDR2

<400>  9

Ala Ile Asn Ser Val Arg His Thr Thr Ser Tyr Ala Asp Ser Val Lys
1               5                   10                  15

Gly

<210>  10
<211>  17
<212>  PRT
<213>  Artificial sequence

<220>
<223>  Nb23 CDR2

<400>  10

Ala Ile Asn Ser Lys Arg His Thr Thr Ser Tyr Ala Asp Ser Val Lys
1               5                   10                  15

Gly

<210>  11
<211>  10
<212>  PRT
<213>  Artificial sequence

<220>
<223>  Nbp75 CDR1

<400>  11

Glu Tyr Pro Ser Ser Ser Thr Ala Met Ala
1               5                   10

<210>  12
<211>  10
<212>  PRT
<213>  Artificial sequence

<220>
<223>  Nbp71 CDR1

<400>  12

Gly Asp Val Pro Glu Asn Gly Tyr Met Ala
1               5                   10

<210>  13
<211>  17
<212>  PRT
<213>  Artificial sequence

<220>
<223>  consensus amino acid sequence of CDR2

<220>
<221>  MISC_FEATURE
<222>  (5)..(5)
<223>  Xaa is a lysine or a valine residue

<220>
<221>  MISC_FEATURE
<222>  (13)..(13)
<223>  Xaa is an aspartate or an asparagine residue

<400>  13

Ala Ile Asn Ser Xaa Arg His Thr Thr Ser Tyr Ala Xaa Ser Val Lys
1               5                   10                  15

Gly

<210>  14
<211>  10

<212>  PRT
<213>  Artificial sequence

<220>
<223>  consensus amino acid sequence of CDR1


<220>
<221>  MISC_FEATURE
<222>  (1)..(1)
<223>  Xaa is a glycine or a glutamate residue

<220>
<221>  MISC_FEATURE
<222>  (2)..(2)
<223>  Xaa is an aspartate or a tyrosine residue

<220>
<221>  MISC_FEATURE
<222>  (3)..(3)
<223>  Xaa is a threonine or a valine or a proline residue

<220>
<221>  MISC_FEATURE
<222>  (4)..(4)
<223>  Xaa is a phenylalanine or a proline or a serine residue

<220>
<221>  MISC_FEATURE
<222>  (5)..(5)
<223>  Xaa is a serine or a glutamate residue

<220>
<221>  MISC_FEATURE
<222>  (6)..(6)
<223>  Xaa is a glycine or an asparagine or a serine residue

<220>
<221>  MISC_FEATURE
<222>  (7)..(7)
<223>  Xaa is a tyrosine or a glycine or a threonine residue

<220>
<221>  MISC_FEATURE
<222>  (8)..(8)
<223>  Xaa is a leucine or a tyrosine or an alanine residue

<220>
<221>  MISC_FEATURE
<222>  (9)..(9)
<223>  Xaa is an alanine or a methionine residue

<400>  14

Xaa Xaa Xaa Xaa Xaa Xaa Xaa Xaa Xaa Ala
1               5                   10


<210>  15
<211>  25
<212>  PRT
<213>  Artificial sequence

<220>
<223>  consensus amino acid sequence of FR1

<220>
<221>  MISC_FEATURE
<222>  (11)..(11)
<223>  Xaa is a serine or an alanine residue

<220>
<221>  MISC_FEATURE
<222>  (14)..(14)
<223>  Xaa is a valine, a proline or an alanine residue

<220>
<221>  MISC_FEATURE
<222>  (19)..(19)
<223>  Xaa is an arginine or a lysine residue

<220>
<221>  MISC_FEATURE
<222>  (20)..(20)
<223>  Xaa is a valine or a leucine residue

<220>
<221>  MISC_FEATURE
<222>  (21)..(21)
<223>  Xaa is an alanine or a serine residue

<220>
<221>  MISC_FEATURE
<222>  (23)..(23)
<223>  Xaa is an alanine, a glutamate or a valine residue

<400>  15

Gln Val Gln Leu Gln Glu Ser Gly Gly Gly Xaa Val Gln Xaa Gly Gly
1               5                   10                  15

Ser Leu Xaa Xaa Xaa Cys Xaa Ala Ser
            20                  25

<210>  16
<211>  14
<212>  PRT
<213>  Artificial sequence

<220>
<223>  consensus amino acid sequence of FR2

<220>
<221>  MISC_FEATURE
<222>  (9)..(9)
<223>  Xaa is a glycine or a glutamate residue

<400>  16

Trp Phe Arg Gln Ala Pro Gly Lys Xaa Arg Glu Gly Val Ala
1               5                   10

<210>  17
<211>  32
<212>  PRT

<213>  Artificial sequence

<220>
<223>  consensus amino acid sequence of FR3


<220>
<221>  MISC_FEATURE
<222>  (12)..(12)
<223>  Xaa is a isoleucine or a methionine residue

<220>
<221>  MISC_FEATURE
<222>  (19)..(19)
<223>  Xaa is a serine or a glycine residue

<400>  17

Arg Phe Thr Ile Ser Lys Asp Asn Ala Asp Asn Xaa Met Tyr Leu Glu
1               5                   10                  15


Met Asn Xaa Leu Lys Pro Glu Asp Thr Ala Ile Tyr Tyr Cys Ala Ala
            20                  25                  30


<210>  18
<211>  11
<212>  PRT
<213>  Artificial sequence

<220>
<223>  amino acid sequence of FR4

<400>  18

Trp Gly Gln Gly Thr Gln Val Thr Val Ser Ser
1               5                   10


<210>  19
<211>  53
<212>  DNA
<213>  Artificial sequence

<220>
<223>  primer #152

<400>  19
ggggacaagt ttgtacaaaa aagcaggctc tatgaaatac ctattgccta cgg          53


<210>  20
<211>  75
<212>  DNA
<213>  Artificial sequence

<220>
<223>  primer #197

<400>  20
ggggaccact ttgtacaaga aagctgggtc ttatttttca aattgaggat gagaccagtg      60

atggtgatgg tggtg                                                       75

<210>  21
<211>  60
<212>  DNA
<213>  Artificial sequence

<220>
<223>  primer #429

<400>  21
atcttaggtc accgtctcct caggcggcgg tagcggcggc ggtatggtga gcaagggcga        60


<210>  22
<211>  53
<212>  DNA
<213>  Artificial sequence

<220>
<223>  primer #430

<400>  22
tactctgaat tctattagtg atgatgatgg tggtgcttgt acagctcgtc cat               53


<210>  23
<211>  50
<212>  DNA
<213>  Artificial sequence

<220>
<223>  primer #153

<400>  23
ggggacaagt ttgtacaaaa aagcaggctc tatgcaggtg cagctgcagg                   50


<210>  24
<211>  49
<212>  DNA
<213>  Artificial sequence

<220>
<223>  primer #150

<400>  24
ggggaccact ttgtacaaga aagctgggtc cttgtacagc tcgtccatg                    49


<210>  25
<211>  28
<212>  DNA
<213>  Artificial sequence

<220>
<223>  primer #397

<400>  25
tcaaatcata tgcaggtgca gctgcagg                                           28


<210>  26
<211>  31
<212>  DNA
<213>  Artificial sequence

<220>
<223>  primer #399

<400>  26
gtgactcgag ttagtgatgg tgatggtgat g                                    31


<210>  27
<211>  21
<212>  DNA
<213>  Artificial sequence

<220>
<223>  RNA1-fwd primer

<400>  27
cccaaaagtc atcgcaatgc t                                               21


<210>  28
<211>  21
<212>  DNA
<213>  Artificial sequence

<220>
<223>  RNA1-rev primer

<400>  28
ggatcaggat atggaaagca c                                               21


<210>  29
<211>  20
<212>  DNA
<213>  Artificial sequence

<220>
<223>  EGFP-fwd primer

<400>  29
tatatcatgg ccgacaagca                                                 20


<210>  30
<211>  20
<212>  DNA
<213>  Artificial sequence

<220>
<223>  EGFP-rev primer

<400>  30
gaactccagc aggaccatgt                                                 20


<210>  31
<211>  20
<212>  DNA
<213>  Artificial sequence

<220>
<223>  P19-fwd primer

<400>  31

tgagatggca attcggtcta                                                    20


<210>  32
<211>  24
<212>  DNA
<213>  Artificial sequence

<220>
<223>  P19-rev primer

<400>  32
tgatacatta ctttccactt cgat                                               24


<210>  33
<211>  22
<212>  DNA
<213>  Artificial sequence

<220>
<223>  CDC2-fwd primer

<400>  33
gtgtagtgta caaggctcgt ga                                                 22


<210>  34
<211>  22
<212>  DNA
<213>  Artificial sequence

<220>
<223>  CDC2-rev primer

<400>  34
tagctgtgct tggtactccc tc                                                 22


<210>  35
<211>  20
<212>  DNA
<213>  Artificial sequence

<220>
<223>  EF1a-fwd primer

<400>  35
gtactgtccc tgttggtcgt                                                    20


<210>  36
<211>  23
<212>  DNA
<213>  Artificial sequence

<220>
<223>  EF1a-rev primer

<400>  36
gtaggtccaa aggtcacaac cat                                                23


<210>  37
<211>  23

<212>  DNA
<213>  Artificial sequence

<220>
<223>  ACT-fwd primer

<400>  37
aaattactgc acttgctcct agc                                                23


<210>  38
<211>  25
<212>  DNA
<213>  Artificial sequence

<220>
<223>  ACT-rev primer

<400>  38
caatccagac actgtatttc ctctc                                              25